# EUROPEAN PATENT SPECIFICATION

(11) **EP 0 520 200 B1**
(45) Date of publication and mention of the grant of the patent: **18.02.1998**
(21) Application number: 92108983.5
(22) Date of filing: 27.05.1992
(51) Int. Cl.: C07D 235/30, A61K 31/415

(54) **Imidazole compounds, their preparation and use**
Imidazol-Verbindungen, Verfahren zu ihrer Herstellung und ihre Anwendung
Composés d'imidazole, leur fabrication et utilisation

(30) Priority: 24.06.1991 US 720021
(43) Date of publication of application: 30.12.1992
(73) Proprietor: NEUROSEARCH A/S, DK-2600 Glostrup (DK)
(72) Inventor: Axelsson, Oskar, S-212 19 Malmö (SE); Thaning, Mikkel, S-222 48 Hjärup (SE); Moldt, Peter, DK-3050 Humlebaek (DK)
(74) Representative: Grünecker, Kinkeldey, Stockmair & Schwanhäusser Anwaltssozietät

(56) References cited:
- EP-A- 0 111 993
- EP-A- 0 477 819
- CHEMICAL ABSTRACTS, vol. 115, 1991, Columbus, Ohio, US; abstract no. 8806f, '1- Phenyl or 1-benzylbenzimidazole derivatives as drugs' & JP-A-0 331 264
- CHEMICAL ABSTRACTS, vol. 111, no. 9, 1989, Columbus, Ohio, US; abstract no. 78007n, 'Preparation of 1-(4-hydroxyphenyl)benzimidazoles as drugs such as antiinflammatories and antihypertensives.' & JP-A-6 400 074

## Description

The present invention relates to therapeutical active compounds and their use as well as to pharmaceutical preparations comprising the compounds. The compounds of the invention possess valuable activity as calcium channel blockers which make them useful in the treatment of anoxia, ischemia, psychosis and migraine for example.

It is well known that an accumulation of calcium (calcium overload) in the brain is seen after anoxia, ischemia, migraine and other hyperactivity periods of the brain, such as after epileptic convulsions. An uncontrolled high concentration of calcium in the brain cells is known to cause most of the degenerative changes connected with above diseases. Therefore compounds which can block the calcium channels of brain cells will be useful in the treatment of anoxia, ischemia, migraine, epilepsia and in the prevention of the degenerative changes connected with the same.

Compounds blocking the socalled L-type calcium channels in the central nervous system (CNS) will be useful for the treatment of the above disorders by directly blocking the calcium uptake in the CNS.

Further, it is well known that the socalled N-type of calcium channels are involved in the regulation of the neurotransmitter release. Compounds blocking the N-type of calcium channels will indirectly and very powerfully prevent calcium overload in CNS after the hyperactivity periods of the brain as described above by inhibiting the enhanced neurotransmitter release seen after such hyperactivity, and especially the neurotoxic enhanced glutamate release after such hyperactivity periods of the CNS. Furthermore, blockers of the N-type of calcium channels will dependent upon the selectivity of the compound in question inhibit the release of various other neurotransmitters such as aspartate, GABA, glycine, dopamine, serotonin and noradrenaline and therefore blockers of N-type of calcium channels may be useful in the treatment of psychosis, Parkinsonism, depression, epilepsia and other convulsive disorders.

From EP-A-0 477 819 benzimidazoles derivatives are known which possess an activity as potassium channel openers. Furthermore, the prior art disclose certain N-substituted benzimidazoles which are described as potent antiviral agents (EP-A-0 111 993), other benzimidazoles are described as antiinflammatories and antihypertensives (Chemical Abstracts, Vol. 111, 1989, abstract No 78007n) and still other related benzimidazoles are described as bactericides, fungicides, virucides, inflammation inhibitors, and rheumatism inhibitors (Chemical Abstracts, Vol. 115, 1991, abstract No 8806f).

It is an object of the present invention to provide compounds being able to block the L-type and/or the N-type calcium channels.

The invention then, inter alia, comprises the following, alone or in combination.

The use of a compound having the formula 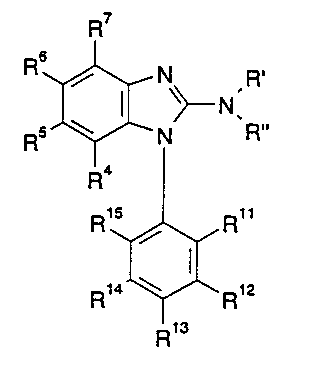 wherein
R' and R'' independently are hydrogen or C₁₋₄-alkyl which may be straight or branched, or R' and R'' together form an alkylene chain of 2-6 carbon atoms;
R⁴, R⁵, R⁶, R⁷ independently of each other are hydrogen, halogen, CF₃, or CN;
R¹¹ is hydrogen, halogen, CF₃, hydroxy or OR^{I} wherein R^{I} is C₁₋₄-alkyl which may be straight or branched;
R¹², R¹³ and R¹⁴ independently are hydrogen, halogen, CF₃, C₁₋₄-alkyl which may be straight or branched, OH, OR¹⁶ wherein R¹⁶ is C₁₋₄-alkyl which may be straight or branched, phenyl, piperidyl, pyrrolidyl, or phenyl which may be substituted one or more times with halogen, CF₃, CN, C₁₋₄-alkyl which may be straight or branched, OH, NO₂, CO₂H, NH₂, OR^{II} wherein R^{II} is C₁₋₄-alkyl which may be straight or branched, CO₂R^{III} wherein R^{III} is C₁₋₄-alkyl which may be straight or branched, NR^{IV}R^{V} wherein R^{IV} and R^{V} independently are hydrogen, C₁₋₆-alkyl which may be straight or branched, acyl, or wherein R^{IV} and R^{V} together form an alkylene chain of 2-6 carbon atoms; R¹⁵ is hydrogen or together with R¹⁴ form an extra benzo ring; or a pharmaceutically acceptable addition salt thereof for the preparation of a medicament useful in the treatment of disorders of a mammal, including a human, responsive to the blockade of N-type and/or L-type of calcium channels,
further the use of a compound as above for the preparation of a medicament useful in the treatment of anoxia, ischemia, migraine, epilepsia, psychosis, Parkinsonism,depression, and for the prevention of the degenerative changes connected with anoxia, ischemia, migraine and epilepsia,
further a compound having the formula 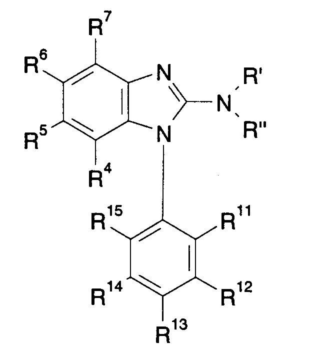 wherein
R' and R'' independently are hydrogen or C₁₋₄-alkyl which may be straight or branched, or R' and R'' together form an alkylene chain of 2-6 carbon atoms;
R⁴, R⁵, R⁶ and R⁷ independently of each other are hydrogen, halogen, CF₃ or CN;
R¹¹ is hydrogen, halogen, CF₃, hydroxy or OR^{I} wherein R^{I} is C₁₋₄-alkyl which may be straight or branched;
two of R¹², R¹³ and R¹⁴ independently are hydrogen, halogen, CF₃, C₁₋₄-alkyl which may be straight or branched, OH, OR¹⁶ wherein R¹⁶ is C₁₋₄-alkyl which may be straight or branched, or phenyl and the last of R¹², R¹³ and R¹⁴ is pyrrolidyl, piperidyl, or phenyl which may be substituted one or more times with halogen, CF₃, CN, C₁₋₄-alkyl which may be straight or branched, OH, NO₂, CO₂H, NH₂, OR^{II} wherein R^{II} is C₁₋₄-alkyl which may be straight or branched, CO₂R^{III} wherein R^{III} is C₁₋₄-alkyl which may be straight or branched, NR^{IV}R^{V} wherein R^{IV} and R^{V} independently are hydrogen, C₁₋₆-alkyl which may be straight or branched, acyl, or wherein R^{IV} and R^{V} together form an alkylene chain of 2-6 carbon atoms; R¹⁵ is hydrogen or together with R¹⁴ form an extra benzo ring; or a pharmaceutically acceptable addition salt thereof,
further the use of a compound as above for the preparation of a medicament useful in the treatment of disorders of a mammal, including a human, responsive to the blockade of N type and/or L-type of calcium channels,
and a compound as above which is 1-(2-hydroxy-5-phenyl-phenyl)-2-amino-benzimidazole,
and a compound as above which is 1-(2-methoxy-5-phenyl-phenyl)-2-amino-benzimidazole,
and a compound as above which is 1-(2-methoxy-5-phenyl-phenyl)-2-amino-5-trifluoromethyl benzimidazole,
and a compound as above which is 1-(3-(1-piperidyl)-phenyl)-5-fluoro-2-amino-benzimidazole,
still further a pharmaceutical composition comprising as active ingredient an effective amount of a compound as above,
further a method of preparing a compound as above comprising
a) reacting a compound having the formula 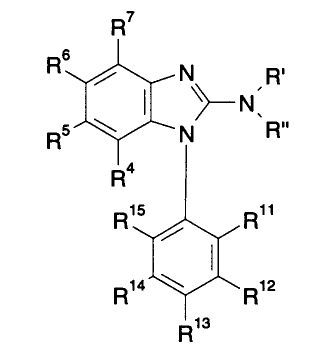 wherein
   R', R'', R⁴, R⁵, R⁶, R⁷, and R¹⁵ have the meanings set forth above and wherein one of R¹², R¹³ and R¹⁴ is iodine and the other of R¹², R¹³ and R¹⁴ have the meanings set forth above, with R¹²-B(OH)₂, R¹³-B(OH)₂ or R¹⁴-B(OH)₂, wherein R¹², R¹³ and R¹⁴ have the meanings set forth above, to form a compound of the invention, or
b) reacting a compound having the formula 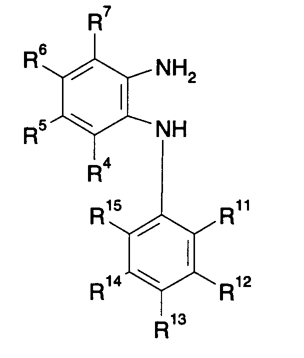 wherein
   R⁴, R⁵, R⁶, R⁷, R¹¹, R¹², R¹³, R¹⁴, and R¹⁵ have the meanings set forth above, with cyanogen bromide, to form a compound of the invention,
   and the method as above wherein 1-(2-hydroxy-5-phenyl-phenyl)-2-amino-benzimidazole is prepared,
   and the method as above wherein 1-(2-methoxy-5-phenyl-phenyl)-2-amino-benzimidazole is prepared,
   and the method as above wherein 1-(2-methoxy-5-phenyl-phenyl)-2-amino-5-trifluoromethyl benzimidazole is prepared,
   and the method as above wherein 1-(3-(1-piperidyl)-phenyl)-5-fluoro-2-amino-benzimidazole is prepared.

Examples of pharmaceutically acceptable addition salts include inorganic and organic acid addition salts such as the hydrochloride, hydrobromide, phosphate, sulphate, citrate, lactate, tartrate, maleate, fumarate, mandelate, oxalate and the acetate.

### Biology

A high influx of calcium from extracelluar compartments into neurons is seen after opening of voltage operated calcium channels. Such opening of calcium channels may be induced by depolarization of neuronal membranes.

A crude synaptosome preparation contains small vesicles surrounded by neuronal membranes, and it is possible to study an opening of the voltage operated calcium channels in such a preparation.

In the below described test influx of ⁴⁵Ca into synaptosomes is studied under depolarized conditions. The effect of test substances on the depolarization induced calcium uptake can thus be studied.

### Test Procedure

The cerebral cortex from a male Wistar rat is homogenized in 20 ml ice cold 0.32M saccharose. In the following steps the temperature is kept at 0°C to 4°C. The homogenate is centrifuged at 1,000 x g for 10 minutes and the supernatant recentrifuged for 20 minutes at 18,000 x g. The obtained pellet is resuspended in 0.32M saccharose (10 ml per g of original tissue).

Aliquots of 0.05 ml of the hereby obtained synaptosome suspension are added to glass tubes containing 0.625 ml of a NaCl buffer (136 mM Nacl, 4 Mm KCl, 0.35 mM CaCl₂, 1.2 mM MgCl₂, 20 mM Tris HCl, 12 mM glucose, pH 7.4) as well as 0.025 ml of different test substances in 48% ethanol. These tubes are pre-incubated for 30 minutes on ice and thereafter for 6 minutes at 37°C.

⁴⁵Ca uptake is initiated by addition to above glass-tubes of 0.4 ml ⁴⁵CaCl₂ (specific activity: 29-39 Ci/g; 0.5 Ci per tube). For depolarized samples the 0.4 ml ⁴⁵CaCl₂ contain KCl (145 mM) and for non-depolarized NaCl (145 mM). The samples are incubated for 15 seconds.

The ⁴⁵Ca uptake is stopped by filtering through glass fibre filters, which are subsequently washed 3 times with an ice cold solution of 145 mM KCl, 7 mM EGTA and 20 mM Tris HCl, pH 7.4 (5.0 ml). The radioactivity on the filters are measured by liquid scintillation spectrometry. Experiments are performed in duplicate.

### Sample preparation

Above test substances are dissolved in for example 10 ml 48% ethanol at a concentration of 0.44 mg/ml. Dilutions are made in ethanol. Test substances are tested at concentrations of 0.1, 0.3, 1, 3, 10 .... µg/ml.

### Results

The test value is given as IC₅₀, that is the concentration in µM of the test substances, which inhibit 50% of the potassium stimulated uptake of ⁴⁵Ca. The uptake in potassium depolarized samples are corrected for basal uptake in non-depolarized samples. The IC₅₀ value is determined from a dose response curve.

The results obtained by testing selected compounds according to the invention are presented in below table

**Table**

| Compound | IC₅₀ (µM) |
|---|---|
| 1-(2,5-dimethoxyphenyl)-2-amino-5-trifluoromethyl-benzimidazole | 0.7 |
| 1-(2-hydroxy-5-chlorophenyl)-5-trifluoromethyl-2-amino-benzimidazole | 2.0 |
| 1-(5-(1-pyrrolidinyl)-phenyl)-2-amino-5-fluoro-benzimidazole | 2.0 |

It has been found (electrophysiological studies using the patch-clamp technique as described by Hamill et al., Pflügers Arch. 391, 85-100 (1981)), that the compounds of the invention block the N-type of calcium channels. Several compounds block the N-type calcium channels in these studies at concentrations from 0.5-10 µM. Examples of such compounds are 1-(2-hydroxy-5-phenyl-phenyl)-2-amino-5-trifluoromethyl-benzimidazole, and 1-(2-methoxy-5-phenyl-phenyl)-2-amino-5-trifluoromethyl-benzimidazole. Therefore the compounds are useful in the treatment of anoxia, ischemia and migraine (see also WO 91/07980).

Further it has been found that the compounds of the invention, for example 1-(2-hydroxy-5-phenyl-phenyl)-2-amino-benzimidazole potently antagonize hypermotility in mice as induced by amphetamine or cocaine. This is in full accordance with the influence of N-type calcium channel blockers on transmitter release in the central nervous system. Therefore the compounds of the invention are useful as anti-psychotics.

### Pharmaceutical Compositions

The compounds of the invention, together with a conventional adjuvant, carrier, or diluent, may be placed into the form of pharmaceutical compositions and unit dosages thereof, and in such form may be employed as solids, such as tablets or filled capsules, or liquids such as solutions, suspensions, emulsions, elixirs, or capsules filled with the same, all for oral use, in the form of suppositories for rectal administration; or in the form of sterile injectable solutions for parenteral (including subcutaneous) use. Such pharmaceutical compositions and unit dosage forms thereof may comprise conventional ingredients in conventional proportions, with or without additional active compounds or principles, and such unit dosage forms may contain any suitable effective amount of the active ingredient commensurate with the intended daily dosage range to be employed. Tablets containing ten (10) milligrams of active ingredient or, more broadly, 0.1 to one hundred (100) milligrams, per tablet, are accordingly suitable representative unit dosage forms.

### Method of Treating

Due to the high degree of activity the compounds of the invention may be administered to a subject, e.g., a living animal body, in need of alleviation, treatment, or amelioration of an indication which is sensitive to the activity or influence of the compounds of the present invention including sensitive to the Ca channel blocking properties of the compounds of the invention. The compounds of the invention are preferably administered in the form of an acid addition salt thereof, concurrently, simultaneously, or together with a pharmaceutically-acceptable carrier or diluent, especially and preferably in the form of a pharmaceutical composition thereof, whether oral, rectal, or parenteral (including subcutaneous) route, in an effective amount. Suitable dosage ranges are 0.1-500 milligrams daily, preferably 1-100 milligrams daily, and especially 1-30 milligrams daily, depending as usual upon the exact mode of administration, form in which administered, the indication toward which the administration is directed, the subject involved and the body weight of the subject involved, and the preferences and experience of the physician or veterinarian in charge.

The following examples will illustrate the invention further.

### Example 1.

**1-(2-Hydroxy-5-trifluoromethylphenyl)-2-amino-5-trifluoromethyl benzimidazole (1P).** To a solution of **2P** (4.17 g, 11.2 mmol) in a mixture of DMF and triethylamine was added cyanogen bromide (1.54 g, 14.6 mmol). The mixture was stirred for 26 h at room temperature and then diluted with water (150 ml), filtered, and neutralized with 2M aqueous sodium hydroxide. The product was filtered off and recrystallized from ethanol. Yield 430 mg, mp >360°C.

In addition to compounds **1A-1AG** also **1-[1-(2-hydroxynaphtyl)]-2-aminobenzimidazole** (mp 210°C) was prepared according to example 1. The starting material in the former case was N-(2-aminophenyl)-1-amino-2-naphthol.

### Example 2.

**1-(2-hydroxy-5-phenyl-phenyl)-2-aminobenzimidazole (1AC).** To a solution of **1H** (100 mg, 0.32 mmol) in dry methylene chloride under nitrogen, was added BBr₃ (0.036 ml, 0.38 mmol). The mixture was stirred at room temperature for 2 h. Water was then added and the pH was adjusted to 7 by the addition of an aqueous solution of sodium carbonate. Extraction with methylene chloride and drying over MgSO₄ was followed by column chromatography on silica gel with methylene chloride/ethanol (25:1) as eluent.
Yield: 40 mg, 0.13 mmol, mp 259-261°C.

### Example 3.

**2-Amino-4,5'-dichloro-2'-hydroxydiphenylamine (20).** A solution of **30** (6.41 g, 21.5 mmol) in a mixture of THF (100 ml) and ethanol (200 ml) was hydrogenated over 5% palladium on charcoal (500 mg) at ambient pressure until the uptake of hydrogen ceased. The mixture was filtered through celite into a flask containing 2.5 ml conc. HCl. Evaporation to dryness gave the product as a dark solid.
Yield: 6.47 g, mp 250-280°C (dec.).

In addition to compounds **2A-2Z** also **N-(2-aminophenyl)-1-amino-2-naphthol** (mp 239-246°C) was prepared according to exam-ple 3. The starting material in the former case was N-(2-nitrophenyl)-1-amino-2-naphthol.

### Example 4.

**2,5-Dimethoxy-2'-nitro-4'-trifluoromethyldiphenylamine (3N).** To a solution of 2,5-dimethoxy aniline (20 g, 130 mmol) in dry DMF under nitrogen, was added 80% NaH in mineral oil (4.31 g, 143 mmol). The mixture was stirred at room temperature for 1 h and then 4-chloro-3-nitrobenzotrifluoride (29.45 g, 130 mmol) was added. After stirring overnight at room temperature the excess sodium hydride was destroyed by the addition of a small amount of water. Dilution with 0.3 M aqueous HCl gave a crystalline product which was filtered off and washed, first with water and then with petroleum ether. The product was dissolved in methylene chloride and filtered through a short column of silica gel. After evaporation of the solvent an orange coloured crystalline solid remained.
Yield 15.33 g, mp 93-95°C.

In addition to compounds **3A-3Z** also **N-(2-nitrophenyl)-1-amino-2-naphthol** (mp 185-188°C) was prepared according to example 3. The starting material in the former case was 3-(2-nitrophenyl)benzo[e]benzoxazole-2-one.

### Example 5.

**4,5'-Bistrifluoromethyl-2'-hydroxy-2-nitrodiphenylamine (3P).** To a solution of **4E** (5.0 g, 12.75 mmol) in dimethoxy ethane was added 38.25 ml 1M aqueous NaOH. The mixture was stirred overnight. Dilution with water and neutralization with 1M HCl was followed by extraction with ether. Evaporation at room temperature gave an orange coloured oil that crystallized upon storage in the freezer.
Yield: 4.01 g, mp 120-123°C.

### Example 6.

**1-(2-Methoxy-5-chlorophenyl)-2-dimethylamino-5-trifluoromethylbenzimidazole.** To a solution of 1Q (o.5 g, 1.37 mmol) in ethanol was added K₂CO₃ (0.38 g, 2.74 mmol) and iodometane (1.94 g, 13.7 mmol). The mixture was refluxed for 20 h, filtered, and evaporated to dryness. The product was purified by chromatography on silica gel with methylene chloride/methanol (9:1) as eluent. Yield 50 mg, mp 230-234°C.

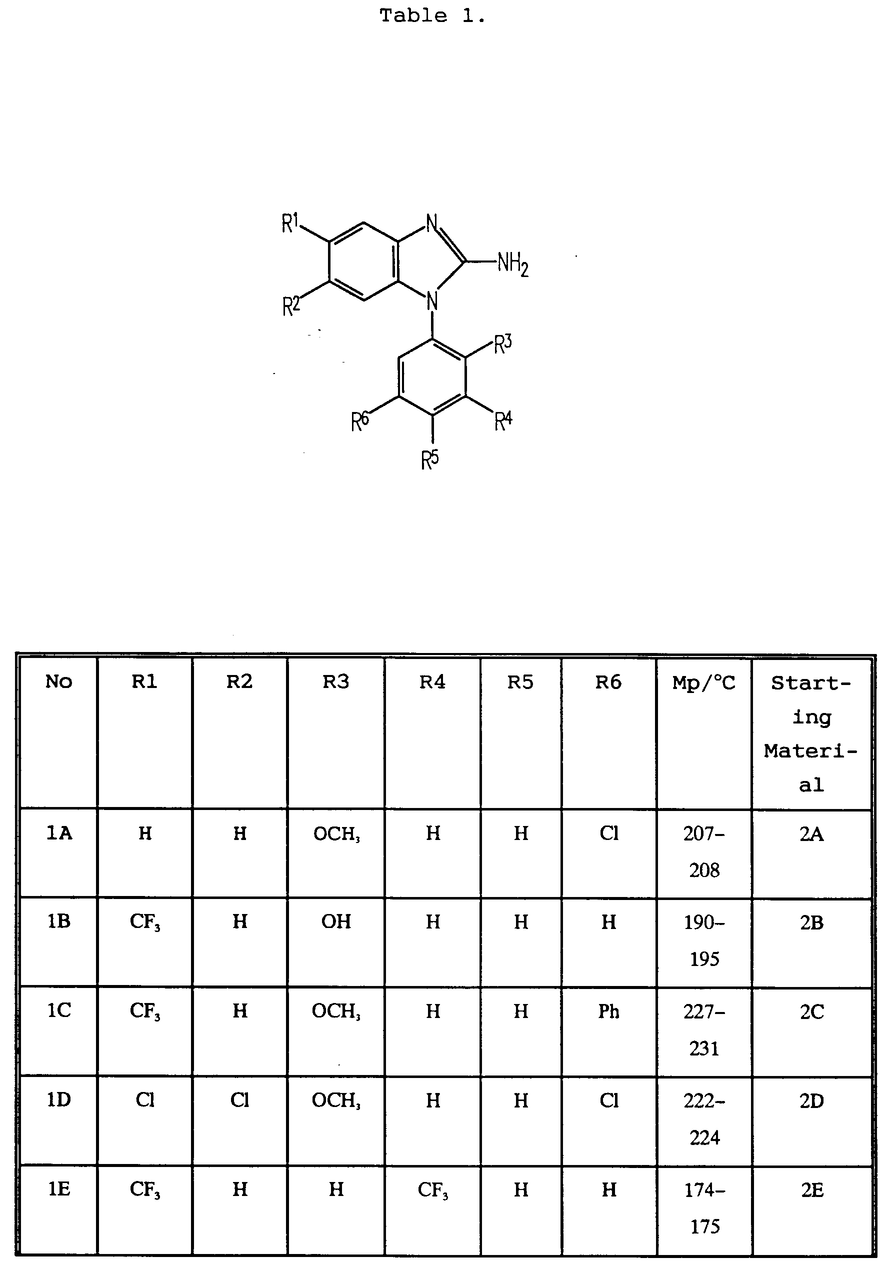

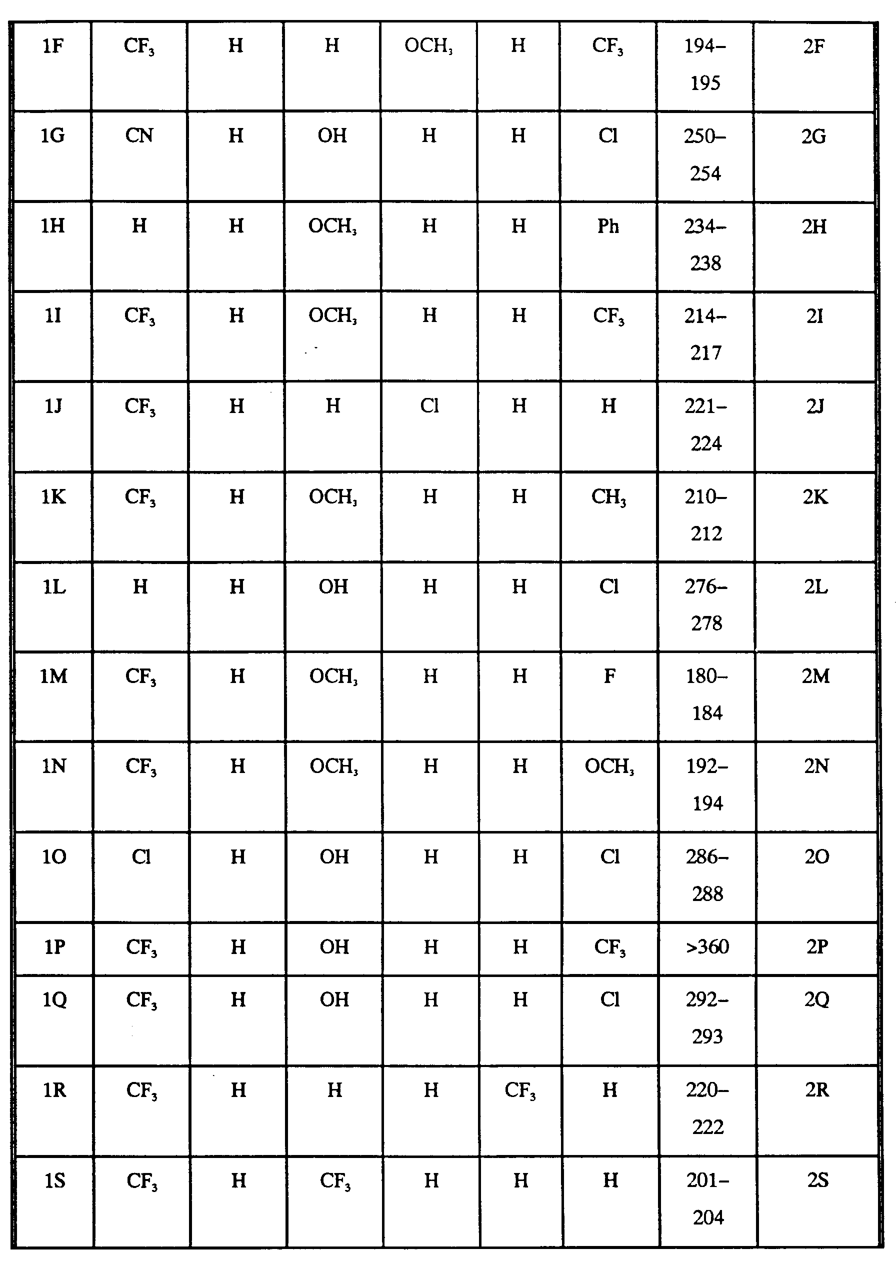

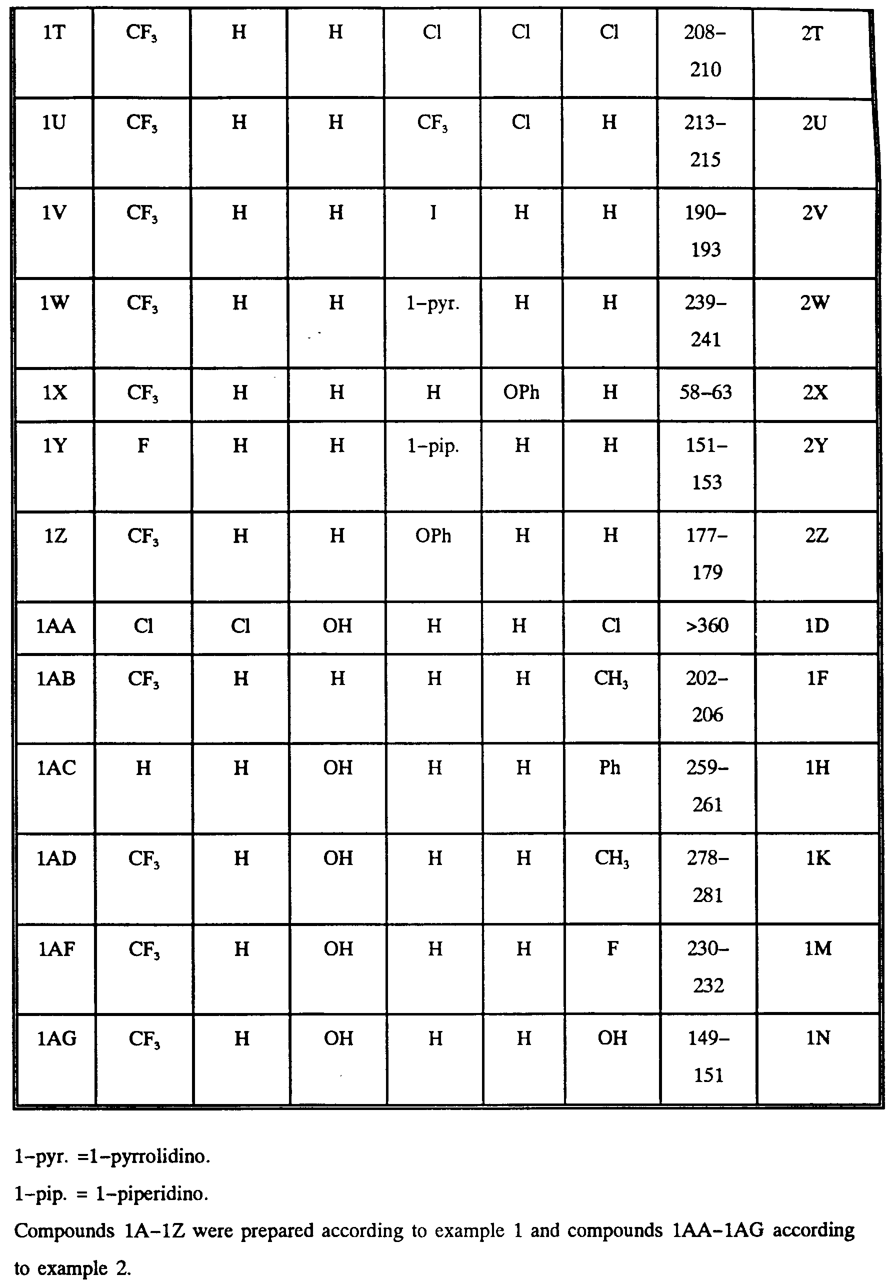

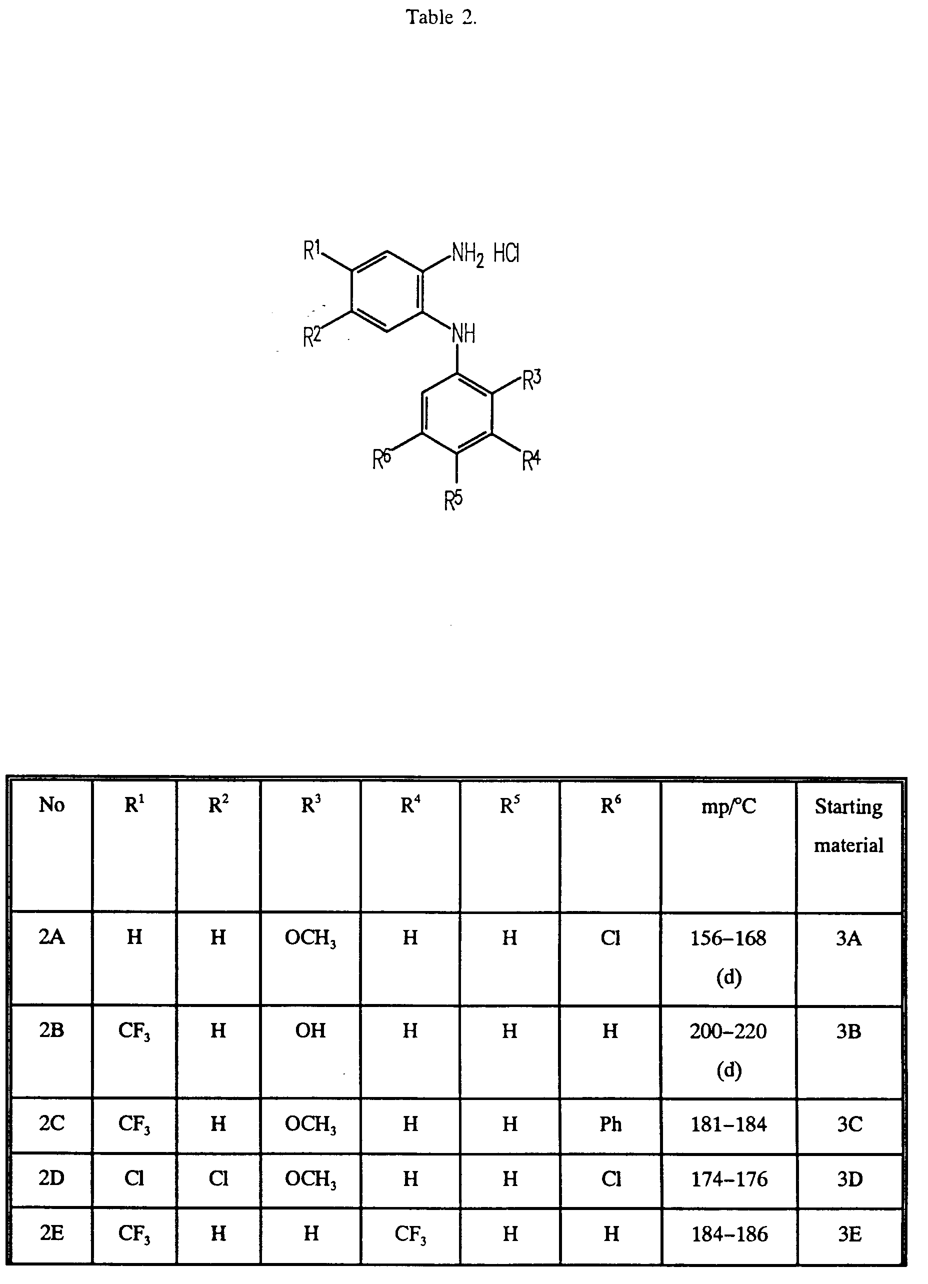

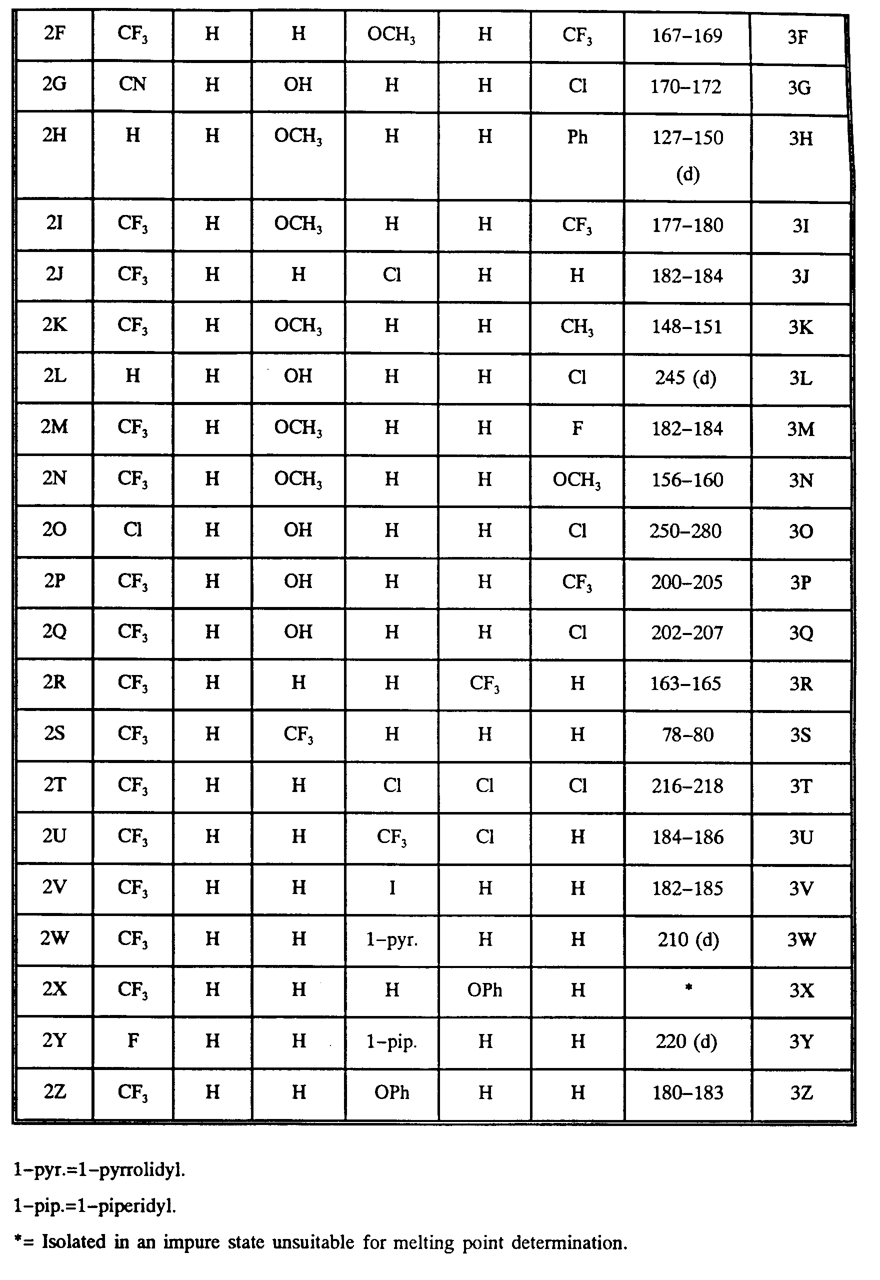

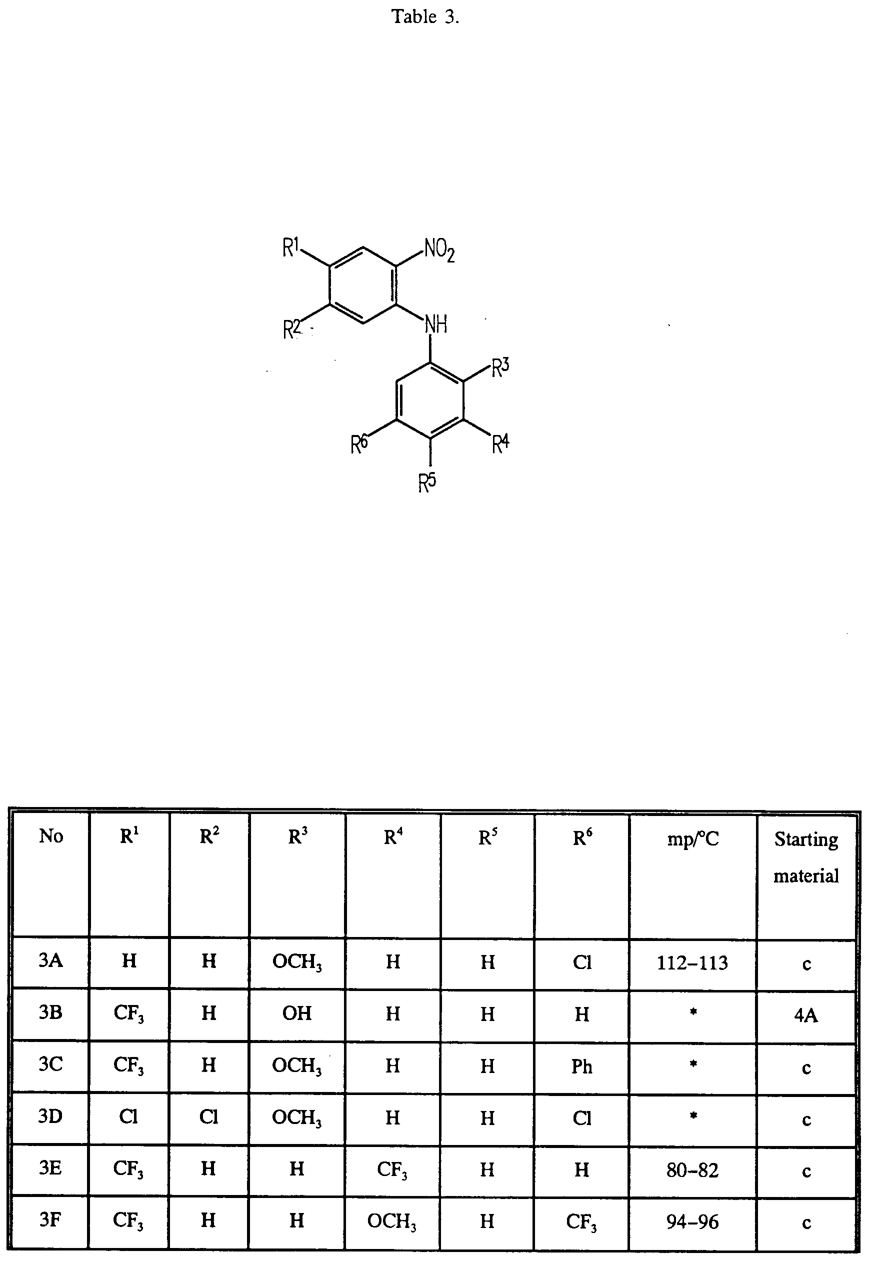

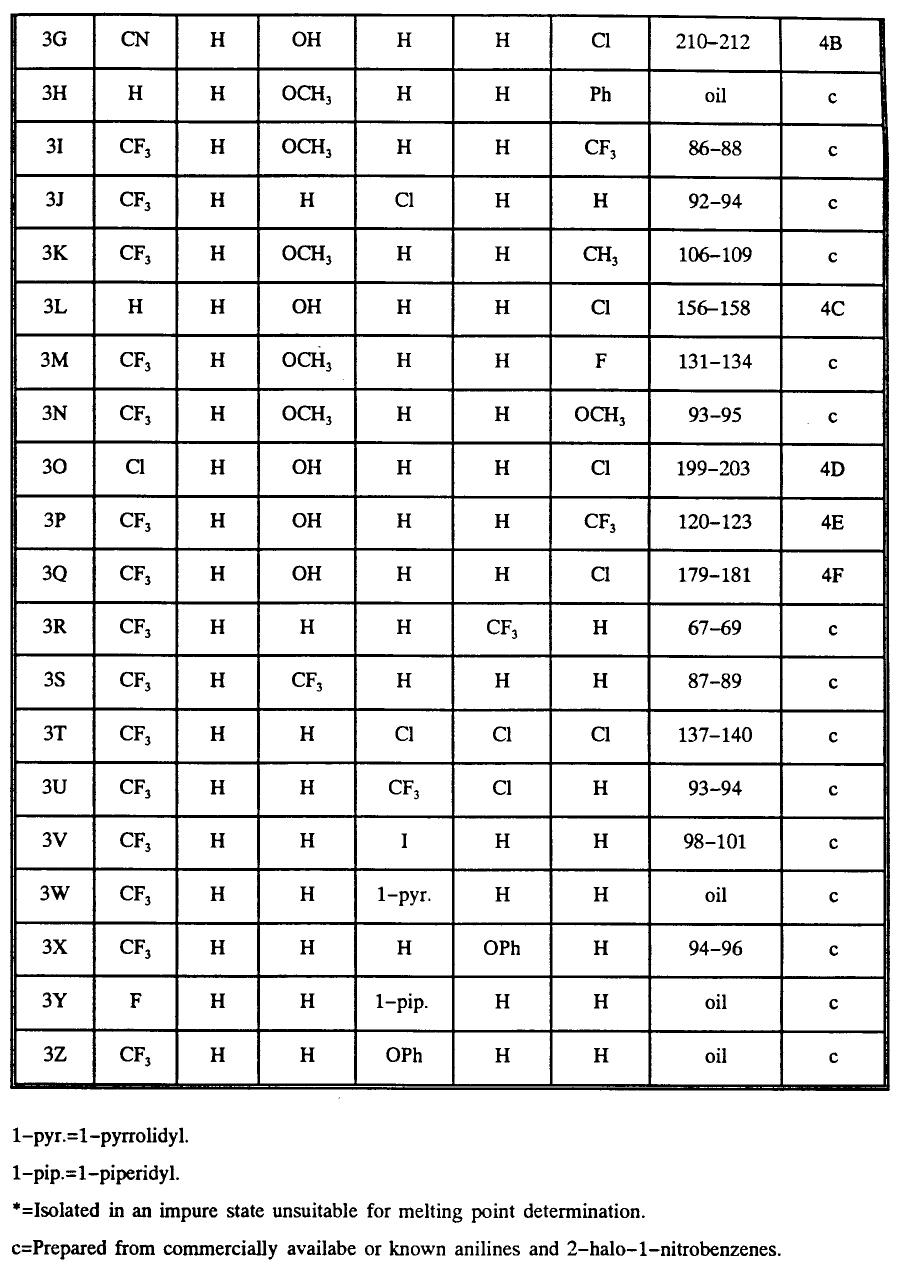

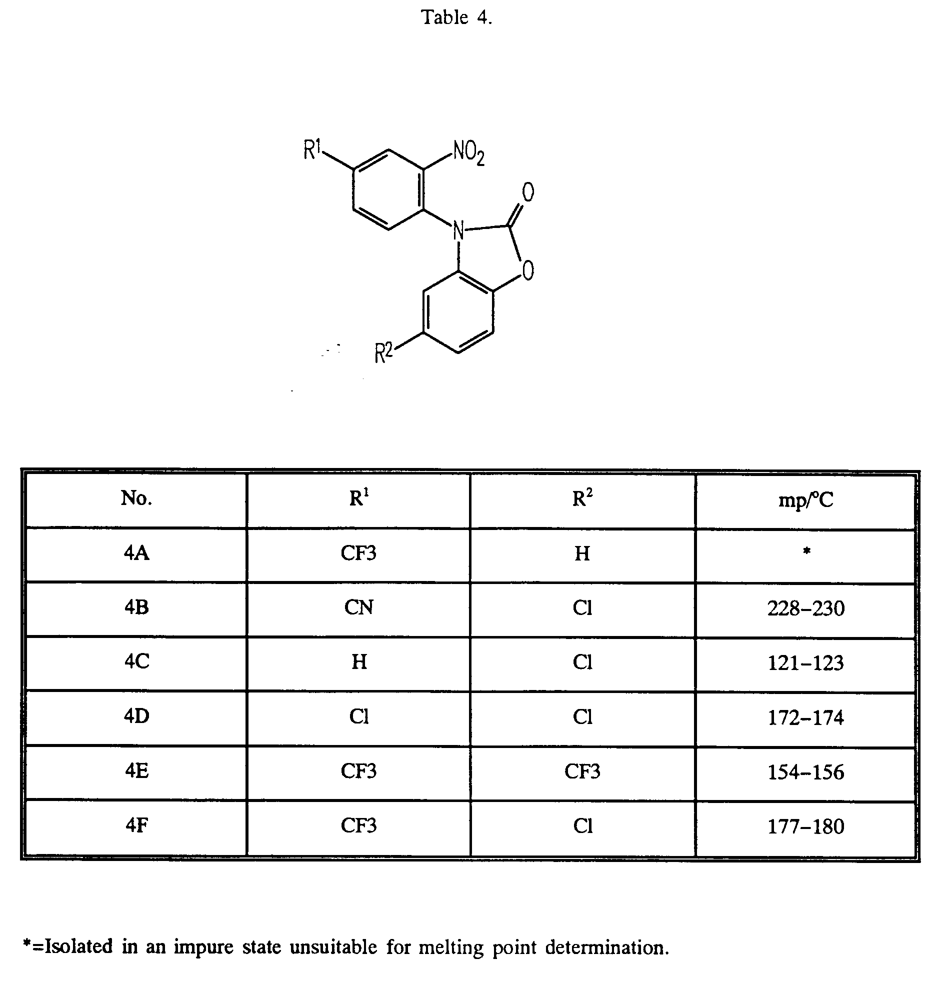

## Claims (Claims for the following Contracting State(s): AT, BE, CH, LI, DE, DK, FR, GB, IT, LU, MC, NL, PT, SE)

1. The use of a compound having the formula 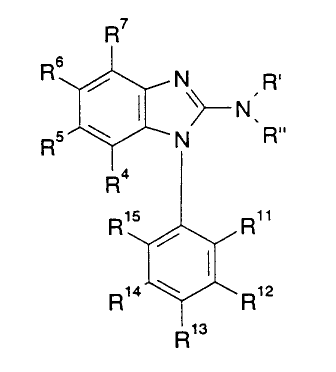 wherein
R' and R'' independently are hydrogen or C₁₋₄-alkyl which may be straight or branched, or R' and R'' together form an alkylene chain of 2-6 carbon atoms;
R⁴, R⁵, R⁶, R⁷ independently of each other are hydrogen, halogen, CF₃, or CN;
R¹¹ is hydrogen, halogen, CF₃, hydroxy or OR^{I} wherein R^{I} is C₁₋₄-alkyl which may be straight or branched;
R¹², R¹³ and R¹⁴ independently are hydrogen, halogen, CF₃, C₁₋₄-alkyl which may be straight or branched, OH, OR¹⁶ wherein R¹⁶ is C₁₋₄-alkyl which may be straight or branched, phenyl, piperidyl, pyrrolidyl, or phenyl which may be substituted one or more times with halogen, CF₃, CN, C₁₋₄-alkyl which may be straight or branched, OH, NO₂, CO₂H, NH₂, OR^{II} wherein R^{II} is C₁₋₄-alkyl which may be straight or branched, CO₂R^{III} wherein R^{III} is C₁₋₄-alkyl which may be straight or branched, NR^{IV}R^{V} wherein R^{IV} and R^{V} independently are hydrogen, C₁₋₆-alkyl which may be straight or branched, acyl, or wherein R^{IV} and R^{V} together form an alkylene chain of 2-6 carbon atoms; R¹⁵ is hydrogen or together with R¹⁴ form an extra benzo ring; or a pharmaceutically acceptable addition salt thereof for the preparation of a medicament useful in the treatment of disorders of a mammal, including a human, responsive to the blockade of N-type and/or L-type of calcium channels.

2. The use of a compound according to claim 1 for the preparation of a medicament useful in the treatment of anoxia, ischemia, migraine, epilepsia, psychosis, Parkinsonism, depression, and the prevention of the degenerative changes connected with anoxia, ischemia, migraine and epilepsia.

3. A compound having the formula 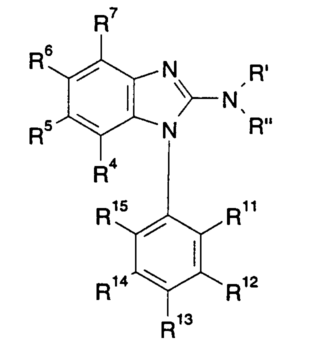 wherein
R' and R'' independently are hydrogen or C₁₋₄-alkyl which may be straight or branched, or R' and R'' together form an alkylene chain of 2-6 carbon atoms;
R⁴, R⁵, R⁶ and R⁷ independently of each other are hydrogen, halogen, CF₃ or CN;
R¹¹ is hydrogen, halogen, CF₃, hydroxy or OR^{I} wherein R^{I} is C₁₋₄-alkyl which may be straight or branched;
two of R¹², R¹³ and R¹⁴ independently are hydrogen, halogen, CF₃, C₁₋₄-alkyl which may be straight or branched, OH, OR¹⁶ wherein R¹⁶ is C₁₋₄-alkyl which may be straight or branched, or phenyl and the last of R¹², R¹³ and R¹⁴ is pyrrolidyl, piperidyl, or phenyl which may be substituted one or more times with halogen, CF₃, CN, C₁₋₄-alkyl which may be straight or branched, OH, NO₂, CO₂H, NH₂, OR^{II} wherein R^{II} is C₁₋₄-alkyl which may be straight or branched, CO₂R^{III} wherein R^{III} is C₁₋₄-alkyl which may be straight or branched, NR^{IV}R^{V} wherein R^{IV} and R^{V} independently are hydrogen, C₁₋₆-alkyl which may be straight or branched, acyl, or wherein R^{IV} and R^{V} together form an alkylene chain of 2-6 carbon atoms; R¹⁵ is hydrogen or together with R¹⁴ form an extra benzo ring; or a pharmaceutically acceptable addition salt thereof.

4. The use of a compound according to claim 3 for the preparation of a medicament useful in the treatment of disorders of a mammal, including a human, responsive to the blockade of N-type and/or L-type of calcium channels.

5. A compound of claim 3 which is 1-(2-hydroxy-5-phenyl-phenyl)-2-amino-benzimidazole.

6. A compound of claim 3 which is 1-(2-methoxy-5-phenyl-phenyl)-2-amino-benzimidazole.

7. A compound of claim 3 which is 1-(2-methoxy-5-phenyl-phenyl)-2-amino-5-trifluoromethyl-benzimidazole.

8. A compound of claim 3 which is 1-(3-(1-piperidyl)-phenyl)-5-fluoro-2-amino-benzimidazole.

9. A pharmaceutical composition comprising as active ingredient an effective amount of a compound of claim 3.

10. A method of preparing a compound according to claim 3 comprising:
a) reacting a compound having the formula 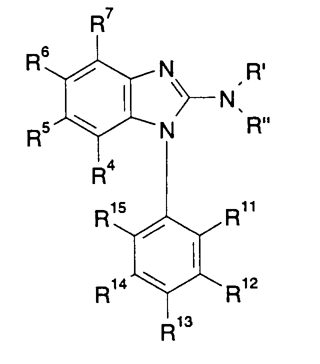 wherein
R', R'', R⁴, R⁵, R⁶, R⁷, and R¹⁵ have the meanings set forth above and wherein one of R¹², R¹³ and R¹⁴ is iodine and the other of R¹², R¹³ and R¹⁴ have the meanings set forth above, with R¹²-B(OH)₂, R¹³-B(OH)₂ or R¹⁴-B(OH)₂, wherein R¹², R¹³ and R¹⁴ have the meanings set forth above, to form a compound of the invention, or
b) reacting a compound having the formula 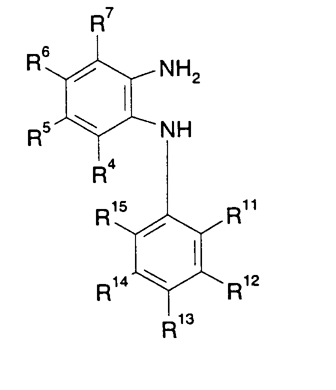 wherein
R⁴, R⁵, R⁶, R⁷, R¹¹, R¹², R¹³, R¹⁴, and R¹⁵ have the meanings set forth above, with cyanogen bromide, to form a compound of the invention.

11. The method of claim 10 wherein 1-(2-hydroxy-5-phenyl-phenyl)-2-amino-benzimidazole is prepared.

12. The method of claim 10 wherein 1-(2-methoxy-5-phenyl-phenyl)-2-amino-benzimidazole is prepared.

13. The method of claim 10 wherein 1-(2-methoxy-5-phenyl-phenyl)-2-amino-5-trifluoromethyl-benzimidazole is prepared.

14. The method of claim 10 wherein 1-(3-(1-piperidyl)-phenyl)-5-fluoro-2-amino-benzimidazole is prepared.

## Claims (Claims for the following Contracting State(s): ES)

1. A compound having the formula 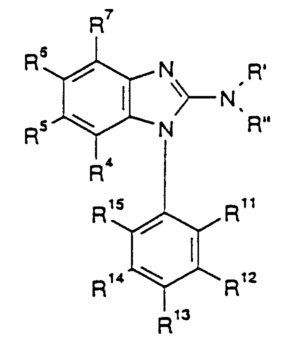 wherein
R' and R'' independently are hydrogen or C₁₋₄-alkyl which may be straight or branched, or R' and R'' together form an alkylene chain of 2-6 carbon atoms;
R⁴, R⁵, R⁶ and R⁷ independently of each other are hydrogen, halogen, CF₃ or CN;
R¹¹ is hydrogen, halogen, CF₃, hydroxy or OR^{I} wherein R^{I} is C₁₋₄-alkyl which may be straight or branched;
two of R¹², R¹³ and R¹⁴ independently are hydrogen, halogen, CF₃, C₁₋₄-alkyl which may be straight or branched, OH, OR¹⁶ wherein R¹⁶ is C₁₋₄-alkyl which may be straight or branched, or phenyl and the last of R¹², R¹³ and R¹⁴ is pyrrolidyl, piperidyl, or phenyl which may be substituted one or more times with halogen, CF₃, CN, C₁₋₄-alkyl which may be straight or branched, OH, NO₂, CO₂H, NH₂, OR^{II} wherein R^{II} is C₁₋₄-alkyl which may be straight or branched, CO₂R^{III} wherein R^{III} is C₁₋₄-alkyl which may be straight or branched, NR^{IV}R^{V} wherein R^{IV} and R^{V} independently are hydrogen, C₁₋₆-alkyl which may be straight or branched, acyl, or wherein R^{IV} and R^{V} together form an alkylene chain of 2-6 carbon atoms; R¹⁵ is hydrogen or together with R¹⁴ form an extra benzo ring; or a pharmaceutically acceptable addition salt thereof.

2. A compound of claim 1 which is 1-(2-hydroxy-5-phenyl-phenyl)-2-amino-benzimidazole.

3. A compound of claim 1 which is 1-(2-methoxy-5-phenyl-phenyl)-2-amino-benzimidazole.

4. A compound of claim 1 which is 1-(2-methroxy-5-phenyl-phenyl)-2-amino-5-trifluoromethyl-benzimidazole.

5. A compound of claim 1 which is 1-(3-(1-piperidyl)-phenyl)-5-fluoro-2-amino-benzimidazole.

6. A method for the preparation of a pharmaceutical composition comprising as active ingredient an effective amount of a compound of claim 1, the method comprising the step of placing the compound together with a conventional adjuvant, carrier or diluent into the form of a pharmaceutical composition.

7. A method of preparing a compound according to claim 1, comprising:
a) reacting a compound having the formula 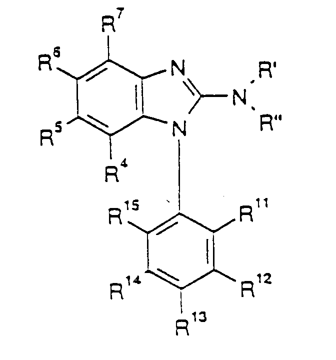 wherein
R', R'', R⁴, R⁵, R⁶, R⁷, and R¹⁵ have the meanings set forth above and wherein one of R¹², R¹³ and R¹⁴ is iodine and the other of R¹², R¹³ and R¹⁴ have the meanings set forth above, with R¹²-B(OH)₂, R¹³-B(OH)₂ or R¹⁴-B(OH)₂, wherein R¹², R¹³ and R¹⁴ have the meanings set forth above, to form a compound of the invention, or
b) reacting a compound having the formula 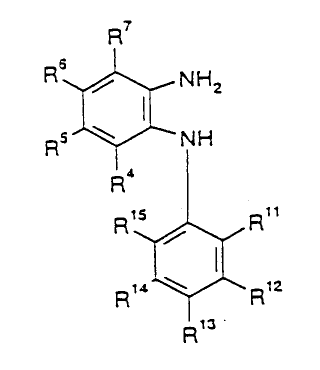 wherein
R⁴, R⁵, R⁶, R⁷, R¹¹, R¹², R¹³, R¹⁴, and R¹⁵ have the meanings set forth above, with cyanogen bromide, to form a compound of the invention.

8. The method of claim 7 wherein 1-(2-hydroxy-5-phenyl-phenyl)-2-amino benzimidazole is prepared.

9. The method of claim 7 wherein 1-(2-methoxy-5-phenyl-phenyl)-2-amino-benzimidazole is prepared.

10. The method of claim 7 wherein 1-(2-methoxy-5-phenyl-phenyl)-2-amino-5-trifluromethyl-benzimidazole is prepared.

11. The method of claim 7 wherein 1-(3-(1-piperidyl)-phenyl)-5-fluoro-2-amino-benzimidazole is prepared.

## Claims (Claims for the following Contracting State(s): GR)

1. The use of a compound having the formula 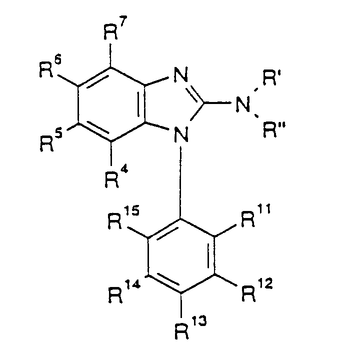 wherein
R' and R'' independently are hydrogen or C₁₋₄-alkyl which may be straight or branched, or R' and R'' together form an alkylene chain of 2-6 carbon atoms;
R⁴, R⁵, R⁶, R⁷ independently of each other are hydrogen, halogen, CF₃, or CN;
R¹¹ is hydrogen, halogen, CF₃, hydroxy or OR^{I} wherein R^{I} is C₁₋₄-alkyl which may be straight or branched;
R¹², R¹³ and R¹⁴ independently are hydrogen, halogen, CF₃, C₁₋₄-alkyl which may be straight or branched, OH, OR¹⁶ wherein R¹⁶ is C₁₋₄-alkyl which may be straight or branched, phenyl, piperidyl, pyrrolidyl, or phenyl which may be substituted one or more times with halogen, CF₃, CN, C₁₋₄-alkyl which may be straight or branched, OH, NO₂, CO₂H, NH₂, OR^{II} wherein R^{II} is C₁₋₄-alkyl which may be straight or branched, CO₂R^{III} wherein R^{III} is C₁₋₄-alkyl which may be straight or branched, NR^{IV}R^{V} wherein R^{IV} and R^{V} independently are hydrogen, C₁₋₆-alkyl which may be straight or branched, acyl, or wherein R^{IV} and R^{V} together form an alkylene chain of 2-6 carbon atoms; R¹⁵ is hydrogen or together with R¹⁴ form an extra benzo ring; or a pharmaceutically acceptable addition salt thereof for the preparation of a medicament useful in the treatment of disorders of a mammal, including a human, responsive to the blockade of N-type and/or L-type of calcium channels.

2. The use of a compound according to claim 1 for the preparation of a medicament useful in the treatment of anoxia, ischemia, migraine, epilepsia psychosis, Parkinsonism, depression, and the prevention of the degenerative changes connected with anoxia, ischemia, migraine and epilepsia.

3. A compound having the formula 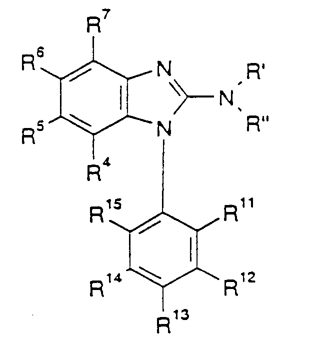 wherein
R' and R'' independently are hydrogen or C₁₋₄-alkyl which may be straight or branched, or R' and R'' together form an alkylene chain of 2-6 carbon atoms;
R⁴, R⁵, R⁶ and R⁷ independently of each other are hydrogen, halogen, CF₃ or CN;
R¹¹ is hydrogen, halogen, CF₃, hydroxy or OR^{I} wherein R^{I} is C₁₋₄-alkyl which may be straight or branched;
two of R¹², R¹³ and R¹⁴ independently are hydrogen, halogen, CF₃, C₁₋₄-alkyl which may be straight or branched, OH, OR¹⁶ wherein R¹⁶ is C₁₋₄-alkyl which may be straight or branched, or phenyl and the last of R¹², R¹³ and R¹⁴ is pyrrolidyl, piperidyl, or phenyl which may be substituted one or more times with halogen, CF₃, CN, C₁₋₄-alkyl which may be straight or branched, OH, NO₂, CO₂H, NH₂, OR^{II} wherein R^{II} is C₁₋₄-alkyl which may be straight or branched, CO₂R^{III} wherein R^{III} is C₁₋₄-alkyl which may be straight or branched, NR^{IV}R^{V} wherein R^{IV} and R^{V} independently are hydrogen, C₁₋₆-alkyl which may be straight or branched, acyl, or wherein R^{IV} and R^{V} together form an alkylene chain of 2-6 carbon atoms; R¹⁵ is hydrogen or together with R¹⁴ form an extra benzo ring; or a pharmaceutically acceptable addition salt thereof.

4. The use of a compound according to claim 3 for the preparation of a medicament useful in the treatment of disorders of a mammal, including a human, responsive to the blockade of N-type and/or L-type of calcium channels.

5. A compound of claim 3 which is 1-(2-hydroxy-5-phenyl-phenyl)-2-amino-benzimidazole.

6. A compound of claim 3 which is 1-(2-methoxy-5-phenyl-phenyl)-2-amino-benzimidazole.

7. A compound of claim 3 which is 1-(2-methoxy-5-phenyl-phenyl)-2-amino-5-trifluoromethyl-benzimidazole.

8. A compound of claim 3 which is 1-(3-(1-piperidyl)-phenyl)-5-fluoro-2-amino-benzimidazole.

9. A method for the preparation of a pharmaceutical composition comprising as active ingredient an effective amount of a compound of claim 3, the method comprising the step of placing the compound together with a conventional adjuvant, carrier or diluent into the form of a pharmaceutical composition.

10. A method of preparing a compound according to claim 3 comprising:
a) reacting a compound having the formula 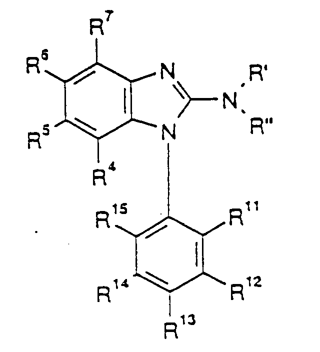 wherein
R', R'', R⁴, R⁵, R⁶, R⁷, and R¹⁵ have the meanings set forth above and wherein one of R¹², R¹³ and R¹⁴ is iodine and the other of R¹², R¹³ and R¹⁴ have the meanings set forth above, with R¹²-B(OH)₂, R¹³-B(OH)₂ or R¹⁴-B(OH)₂ wherein R¹², R¹³ and R¹⁴ have the meanings set forth above, to form a compound of the invention, or
b) reacting a compound having the formula 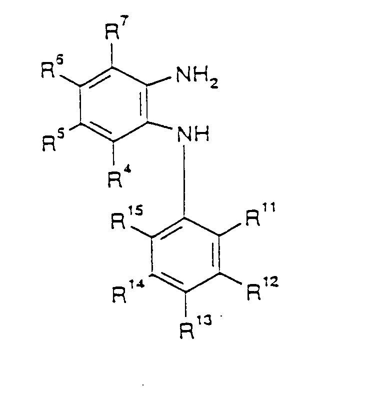 wherein
R⁴, R⁵, R⁶, R⁷, R¹¹, R¹², R¹³, R¹⁴, and R¹⁵ have the meanings set forth above, with cyanogen bromide, to form a compound of the invention.

11. The method of claim 10 wherein 1-(2-hydroxy-5-phenyl-phenyl)-2-amino benzimidazole is prepared.

12. The method of claim 10 wherein 1-(2-methoxy-5-phenyl-phenyl)-2-amino-benzimidazole is prepared.

13. The method of claim 10 wherein 1-(2-methoxy-5-phenyl-phenyl)-2-amino-5-trifluromethyl-benzimidazole is prepared.

14. The method of claim 10 wherein 1-(3-(1-piperidyl)-phenyl)-5-fluoro-2-amino-benzimidazole is prepared.

## Patentansprüche (Patentansprüche für folgende(n) Vertragsstaat(en): AT, BE, CH, LI, DE, DK, FR, GB, IT, LU, MC, NL, PT, SE)

1. Verwendung einer Verbindung der Formel 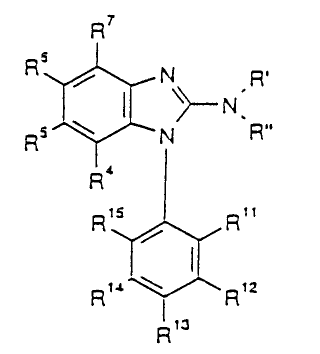 worin
R' und R'' unabhängig voneinander Wasserstoff oder ein C₁₋₄-Alkyl bedeuten, das linear oder verzweigt sein kann oder R' und R'' bilden zusammen eine Alkylen-Kette mit 2-6 Kohlenstoffatomen; R⁴, R⁵, R⁶ und R⁷ sind unabhängig voneinander Wasserstoff, Halogen, CF₃ oder CN; R¹¹ ist Wasserstoff, Halogen, CF₃, Hydroxy oder OR^{'} wobei R^{'} ein C₁₋₄-Alkyl ist, das linear oder verzweigt sein kann; R¹², R¹³ und R¹⁴ bedeuten unabhängig voneinander Wasserstoff, Halogen, CF₃, C₁₋₄-Alkyl, das linear oder verzweigt sein kann, OH, OR¹⁶, wobei R¹⁶ ein C₁₋₄-Alkyl ist, das linear oder verzweigt sein kann, Phenyl, Piperidyl, Pyrrolidyl oder Phenyl, das einfach oder mehrfach mit Halogen, CF₃, CN, C₁₋₄-Alkyl, das linear oder verzweigt sein kann, OH, NO₂, CO₂H, NH₂, OR'', wobei R'' ein C₁₋₄-Alkyl ist, das linear oder verzweigt sein kann, CO₂R''', wobei R''' ein C₁₋₄-Alkyl ist, das linear oder verzweigt sein kann, NR^{IV}R^{V}, wobei R^{IV} und R^{V} unabhängig voneinander Wasserstoff, C₁₋₆-Alkyl, das linear oder verzweigt sein kann, Acyl bedeuten kann oder wobei R^{IV} und R^{V} miteinander eine Alkylenkette mit 2-6 Kohlenstoffatomen bilden, substituiert sein kann; R¹⁵ ist Wasserstoff oder bildet zusammen mit R¹⁴ einen Extra-Benzoring; oder ein pharmazeutisch akzeptables Additionssalz davon zur Herstellung eines Medikaments, das für die Behandlung von Störungen eines Säugetiers, einschließlich eines Menschen, die auf eine Blockade der N-Typ- und/oder L-Typ-Calciumkanäle reagieren, geeignet ist.

2. Verwendung einer Verbindung nach Anspruch 1 für die Herstellung eines Medikaments, das zur Behandlung von Anoxie, Ischämie, Migräne, Epilepsie, Psychose, Parkinson-Krankheit, Depression und der Verhinderung degenerativer Veränderungen in Verbindung mit Anoxie, Ischämie, Migräne und Epilepsie geeignet ist.

3. Verbindung der Formel 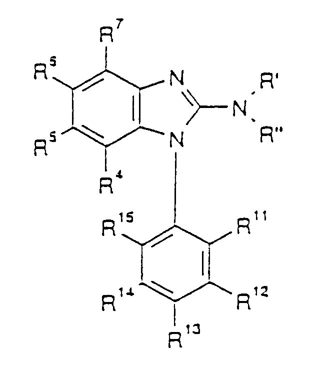 worin
R' und R'' unabhängig voneinander Wasserstoff oder ein C₁₋₄-Alkyl bedeuten, das linear oder verzweigt sein kann oder R' und R'' bilden zusammen eine Alkylen-Kette mit 2-6 Kohlenstoffatomen; R⁴, R⁵, R⁶ und R⁷ sind unabhängig voneinander Wasserstoff, Halogen, CF₃ oder CN; R¹¹ ist Wasserstoff, Halogen, CF₃, Hydroxy oder OR', wobei R' ein C₁₋₄-Alkyl ist, das linear oder verzweigt sein kann; zwei der Reste R¹², R¹³ und R¹⁴ bedeuten unabhängig voneinander Wasserstoff, Halogen, CF₃, C₁₋₄-Alkyl, das linear oder verzweigt sein kann, OH, OR¹⁶, wobei R¹⁶ ein C₁₋₄-Alkyl ist, das linear oder verzweigt sein kann oder Phenyl und der letzte der Reste R¹², R¹³ und R¹⁴ ist Pyrrolidyl, Piperidyl oder Phenyl, das einfach oder mehrfach mit Halogen, CF₃, CN, C₁₋₄-Alkyl, das linear oder verzweigt sein kann, OH, NO₂, CO₂H, NH₂, OR'', wobei R'' ein C₁₋₄-Alkyl ist, das linear oder verzweigt sein kann, CO₂R''', wobei 12''' ein C₁₋₄-Alkyl ist, das linear oder verzweigt sein kann, NR^{IV}R^{V}, wobei R^{IV} und R^{V} unabhängig Wasserstoff sind, C₁₋₆-Alkyl, das linear oder verzweigt sein kann, Acyl bedeuten kann oder wobei R^{IV} und R^{V} miteinander eine Alkylenkette mit 2-6 Kohlenstoffatomen bilden, substituiert sein kann; R¹⁵ ist Wasserstoff oder bildet zusammen mit R¹⁴ einen Extra-Benzoring; oder ein pharmazeutisch akzeptables Additionssalz davon.

4. Verwendung einer Verbindung nach Anspruch 3 für die Herstellung eines Medikaments, das zur Behandlung von Störungen eines Säugers, einschließlich eines Menschen, geeignet ist, die auf eine Blockade der N-Typ- oder L-Typ- Calciumkanäle reagieren.

5. Verbindung nach Anspruch 3, die 1-(2-Hydroxy-5-phenyl-phenyl)-2-amino-benzimidazol ist.

6. Verbindung nach Anspruch 3, die 1-(2-Methoxy-5-phenyl-phenyl)-2-amino-benzimidazol ist.

7. Verbindung nach Anspruch 3, die 1-(2-Methoxy-5-phenyl-phenyl)-2-amino-5-trifluormethyl-benzimidazol ist.

8. Verbindung nach Anspruch 3, die 1-(3-(1-Piperidyl)-phenyl)-5-fluor-2-amino-benzimidazol ist.

9. Pharmazeutische Zusammensetzung, umfassend als aktiven Bestandteil eine wirksame Menge einer Verbindung nach Anspruch 3.

10. Verfahren zur Herstellung einer Verbindung nach Anspruch 3, umfassend:
a) Umsetzen einer Verbindung der Formel 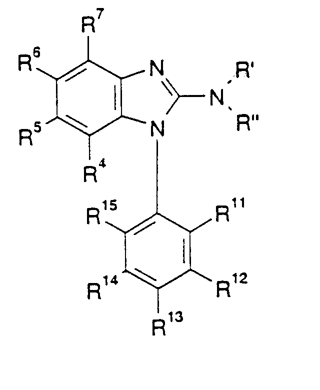 worin
R^{'}, R''; R⁴, R⁵, R⁶, R⁷ und R¹⁵ die oben definierten Bedeutungen haben und wobei einer der Reste R¹², R¹³ und R¹⁴ Iod bedeutet und die anderen Reste R¹², R¹³ und R¹⁴ die oben dargestellten Bedeutungen haben,
mit R¹²-B(OH)₂, R¹³-B(OH)₂ oder R¹⁴-B(OH)₂, wobei R¹², R¹³ und R¹⁴ die oben definierten Bedeutungen haben, zur Bildung einer Verbindung der Erfindung oder
b) Umsetzung einer Verbindung der Formel 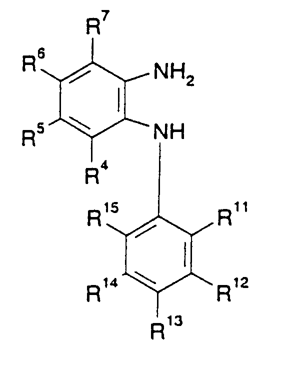 worin
R⁴, R⁵, R⁶, R⁷, R¹¹, R¹², R¹³, R¹⁴ und R¹⁵ die oben definierten Bedeutungen haben,
mit Cyanogenbromid zur Bildung einer Verbindung der Erfindung.

11. Verfahren nach Anspruch 10, wobei 1-(2-Hydroxy-5-phenyl-phenyl)-2-amino-benzimidazol hergestellt wird.

12. Verfahren nach Anspruch 10, wobei 1-(2-Methoxy-5-phenyl-phenyl)-2-amino-benzimidazol hergestellt wird.

13. Verfahren nach Anspruch 10, wobei 1-(2-Methoxy-5-phenyl-phenyl)-2-amino-5-trifluormethyl-benzimidazol hergestellt wird.

14. Verfahren nach Anspruch 10, wobei 1-(3-(1-Piperidyl)-phenyl)-5-fluor-2-amino-benzimidazol hergestellt wird.

## Patentansprüche (Patentansprüche für folgende(n) Vertragsstaat(en): ES)

1. Verbindung der Formel 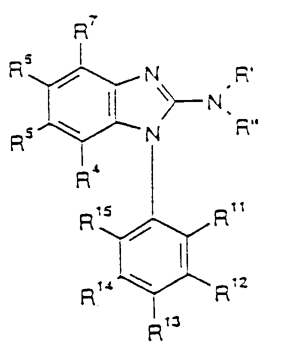 worin
R' und R'' unabhängig voneinander Wasserstoff oder ein C₁₋₄-Alkyl bedeuten, das linear oder verzweigt sein kann oder R' und R'' bilden zusammen eine Alkylen-Kette mit 2-6 Kohlenstoffatomen; R⁴, R⁵, R⁶ und R⁷ sind unabhängig voneinander Wasserstoff, Halogen, CF₃ oder CN; R¹¹ ist Wasserstoff, Halogen, CF₃, Hydroxy oder OR^{'}, wobei R' ein C₁₋₄-Alkyl ist, das linear oder verzweigt sein kann; zwei der Reste R¹², R¹³ und R¹⁴ bedeuten unabhängig voneinander Wasserstoff, Halogen, CF₃, C₁₋₄-Alkyl, das linear oder verzweigt sein kann, OH, OR¹⁶, wobei R¹⁶ ein C₁₋₄-Alkyl ist, das linear oder verzweigt sein kann oder Phenyl, und der letzte der Reste R¹²,R¹³ und R¹⁴ ist Pyrrolidyl, Piperidyl oder Phenyl, das einfach oder mehrfach mit Halogen, CF₃, CN, C₁₋₄-Alkyl, das linear oder verzweigt sein kann, OH, NO₂, CO₂H, NH₂, OR'', wobei R'' ein C₁₋₄-Alkyl ist, das linear oder verzweigt sein kann, CO₂R''', wobei R''' ein C₁₋₄-Alkyl ist, das linear oder verzweigt sein kann, NR^{IV}R^{V}, wobei R^{IV} und R^{V} unabhängig voneinander Wasserstoff, C₁₋₆-Alkyl, das linear oder verzweigt sein kann, Acyl bedeuten kann oder wobei R^{IV} und R^{V} miteinander eine Alkylenkette mit 2-6 Kohlenstoffatomen bilden, substituiert sein kann; R¹⁵ ist Wasserstoff oder bildet zusammen mit R¹⁴ einen Extra-Benzoring; oder ein pharmazeutisch akzeptables Additionssalz davon.

2. Verbindung nach Anspruch 1, die 1-(2-Hydroxy-5-phenyl-phenyl)-2-amino-benzimidazol ist.

3. Verbindung nach Anspruch 1, die 1-(2-Methoxy-5-phenyl-phenyl)-2-amino-benzimidazol ist.

4. Verbindung nach Anspruch 1, die 1-(2-Methoxy-5-phenyl-phenyl)-2-amino-5-trifluormethyl-benzimidazol ist.

5. Verbindung nach Anspruch 1, die 1-(3-(1-Piperidyl)-phenyl)-5-fluor-2-amino-benzimidazol ist.

6. Verfahren zur Herstellung einer pharmazeutischen Zusammensetzung, umfassend als aktiven Bestandteil eine wirksame Menge der Verbindung des Anspruchs 1, wobei das Verfahren den Schritt der Zusammenbringung der Verbindung mit einem konventionellen Adjuvans, Träger oder Verdünnungsmittel in Form einer pharmazeutischen Zusammensetzung umfaßt.

7. Verfahren zur Herstellung einer Verbindung nach Anspruch 1, umfassend:
a) Umsetzen einer Verbindung der Formel 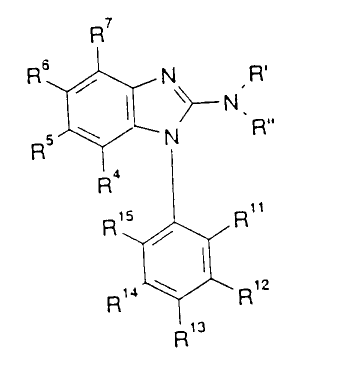 worin
R^{'}, R'', R⁴, R⁵, R⁶, R⁷ und R¹⁵ die oben definierten Bedeutungen haben und wobei einer der Reste R¹², R¹³ und R¹⁴ Iod bedeutet und die anderen Reste R¹², R¹³ und R¹⁴ die oben dargestellten Bedeutungen haben,
mit R¹²-B(OH)₂, R¹³-B(OH)₂ oder R¹⁴-B(OH)₂, wobei R¹², R¹³ und R¹⁴ die oben definierten Bedeutungen haben, zur Bildung einer Verbindung der Erfindung oder
b) Umsetzung einer Verbindung der Formel 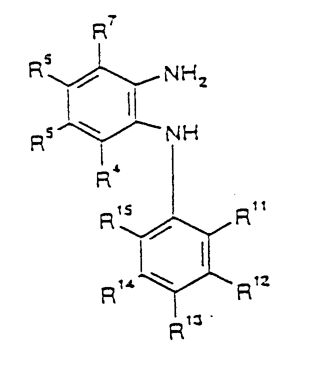 worin
R⁴, R⁵, R⁶, R⁷, R¹¹, R¹², R¹³, R¹⁴ und R¹⁵ die oben definierten Bedeutungen haben,
mit Cyanogenbromid zur Bildung einer Verbindung der Erfindung.

8. Verfahren nach Anspruch 7, wobei 1-(2-Hydroxy-5-phenyl-phenyl)-2-amino-benzimidazol hergestellt wird.

9. Verfahren nach Anspruch 7, wobei 1-(2-Methoxy-5-phenyl-phenyl)-2-amino-benzimidazol hergestellt wird.

10. Verfahren nach Anspruch 7, wobei 1-(2-Methoxy-5-phenyl-phenyl)-2-amino-5-trifluormethyl-benzimidazol hergestellt wird.

11. Verfahren nach Anspruch 7, wobei 1-(3-(1-Piperidyl)-phenyl)-5-fluor-2-amino-benzimidazol hergestellt wird.

## Patentansprüche (Patentansprüche für folgende(n) Vertragsstaat(en): GR)

1. Verwendung einer Verbindung der Formel 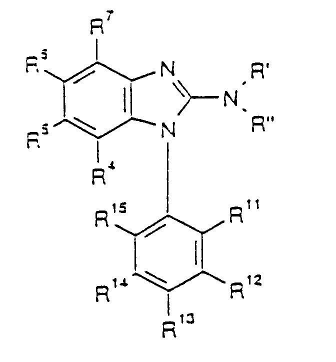 worin
R' und R'' unabhängig voneinander Wasserstoff oder ein C₁₋₄-Alkyl bedeuten, das linear oder verzweigt sein kann oder R' und R'' bilden zusammen eine Alkylen-Kette mit 2-6 Kohlenstoffatomen; R⁴, R⁵, R⁶ und R⁷ sind unabhängig voneinander Wasserstoff, Halogen, CF₃ oder CN; R¹¹ ist Wasserstoff, Halogen, CF₃, Hydroxy oder OR^{'}, wobei R' ein C₁₋₄-Alkyl ist, das linear oder verzweigt sein kann; R¹², R¹³ und R¹⁴ bedeuten unabhängig voneinander Wasserstoff, Halogen, CF₃, C₁₋₄-Alkyl, das linear oder verzweigt sein kann, OH, OR¹⁶, wobei R¹⁶ ein C₁₋₄-Alkyl ist, das linear oder verzweigt sein kann, Phenyl, Piperidyl, Pyrrolidyl oder Phenyl, das einfach oder mehrfach mit Halogen, CF₃, CN, C₁₋₄-Alkyl, das linear oder verzweigt sein kann, OH, NO₂, CO₂H, NH₂, OR'', wobei R'' ein C₁₋₄-Alkyl ist, das linear oder verzweigt sein kann, CO₂R''', wobei R''' ein C₁₋₄-Alkyl ist, das linear oder verzweigt sein kann, NR^{IV}R^{V}, wobei R^{IV} und R^{V} unabhängig voneinander Wasserstoff, C₁₋₆-Alkyl, das linear oder verzweigt sein kann, Acyl bedeuten kann oder wobei R^{IV} und R^{V} miteinander eine Alkylenkette mit 2-6 Kohlenstoffatomen bilden substituiert sein kann; R¹⁵ ist Wasserstoff oder bildet zusammen mit R¹⁴ einen Extra-Benzoring; oder ein pharmazeutisch akzeptables Additionssalz davon zur Herstellung eines Medikaments, das für die Behandlung von Störungen eines Säugetiers, einschließlich eines Menschen, die auf eine Blockade der N-Typ- und/oder L-Typ-Calciumkanäle reagieren, geeignet ist.

2. Verwendung einer Verbindung nach Anspruch 1 für die Herstellung eines Medikaments, das zur Behandlung von Anoxie, Ischämie, Migräne, Epilepsie, Psychose, Parkinson-Krankheit, Depression und der Verhinderung degenerativer Veränderungen in Verbindung mit Anoxie, Ischäme, Migräne und Epilepsie geeignet ist.

3. Verbindung der Formel 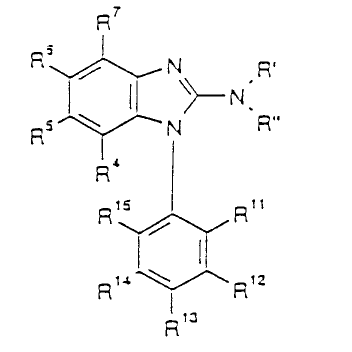 worin
R' und R'' unabhängig voneinander Wasserstoff oder ein C₁₋₄-Alkyl bedeuten, das linear oder verzweigt sein kann oder R' und R'' bilden zusammen eine Alkylen-Kette mit 2-6 Kohlenstoffatomen; R⁴, R⁵, R⁶ und R⁷ sind unabhängig voneinander Wasserstoff, Halogen, CF₃ oder CN; R¹¹ ist Wasserstoff, Halogen, CF₃, Hydroxy oder OR', wobei R' ein C₁₋₄-Alkyl ist, das linear oder verzweigt sein kann; zwei der Reste R¹², R¹³ und R¹⁴ bedeuten unabhängig voneinander Wasserstoff, Halogen, CF₃, C₁₋₄-Alkyl, das linear oder verzweigt sein kann, OH, OR¹⁶, wobei R¹⁶ ein C₁₋₄-Alkyl ist, das linear oder verzweigt sein kann oder Phenyl und der letzte der Reste R¹², R¹³ und R¹⁴ ist Pyrrolidyl, Piperidyl oder Phenyl, das einfach oder mehrfach mit Halogen, CF₃, CN, C₁₋₄-Alkyl, das linear oder verzweigt sein kann, OH, NO₂, CO₂H, NH₂, OR'', wobei R'' ein C₁₋₄-Alkyl ist, das linear oder verzweigt sein kann, CO₂R''', wobei R''' ein C₁₋₄-Alkyl ist, das linear oder verzweigt sein kann, NR^{IV}R^{V}, wobei R^{IV} und R^{V} unabhängig voneinander Wasserstoff, C₁₋₆-Alkyl, das linear oder verzweigt sein kann, Acyl bedeuten kann oder wobei R^{IV} und R^{V} miteinander eine Alkylenkette mit 2-6 Kohlenstoffatomen bilden, substituiert sein kann; R¹⁵ ist Wasserstoff oder bildet zusammen mit R¹⁴ einen Extra-Benzoring; oder ein pharmazeutisch akzeptables Additionssalz davon.

4. Verwendung einer Verbindung nach Anspruch 3 für die Herstellung eines Medikaments, das zur Behandlung von Störungen eines Säugers, einschließlich eines Menschen, geeignet ist, die auf eine Blockade der N-Typ- oder L-Typ- Calciumkanäle reagieren.

5. Verbindung nach Anspruch 3, die 1-(2-Hydroxy-5-phenyl-phenyl)-2-amino-benzimidazol ist.

6. Verbindung nach Anspruch 3, die 1-(2-Methoxy-5-phenyl-phenyl)-2-amino-benzimidazol ist.

7. Verbindung nach Anspruch 3, die 1-(2-Methoxy-5-phenyl-phenyl)-2-amino-5-trifluormethyl-benzimidazol ist.

8. Verbindung nach Anspruch 3, die 1-(3-(1-Piperidyl)-phenyl)-5-fluor-2-amino-benzimidazol ist.

9. Verfahren zur Herstellung einer pharmazeutischen Zusammensetzung, umfassend als aktiven Bestandteil eine wirksame Menge der Verbindung des Anspruchs 3, wobei das Verfahren den Schritt der Zusammenbringung der Verbindung mit einem konventionellen Adjuvans, Träger oder Verdünnungsmittel in Form einer pharmazeutischen Zusammensetzung umfaßt.

10. Verfahren zur Herstellung einer Verbindung nach Anspruch 3, umfassend:
a) Umsetzen einer Verbindung der Formel 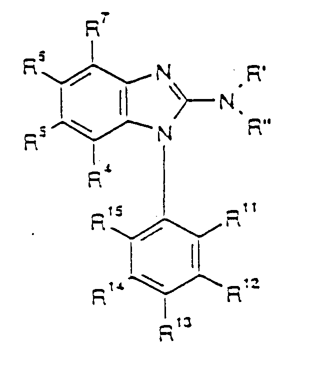 worin
R^{'}, R'', R⁴, R⁵, R⁶, R⁷ und R¹⁵ die oben definierten Bedeutungen haben und wobei einer der Reste R¹², R¹³ und R¹⁴ Iod bedeutet und die anderen Reste R¹², R¹³ und R¹⁴ die oben dargestellten Bedeutungen haben,
mit R¹²-B(OH)₂, R¹³-B(OH)₂ oder R¹⁴-B(OH)₂, wobei R¹², R¹³ und R¹⁴ die oben definierten Bedeutungen haben, zur Bildung einer Verbindung der Erfindung oder
b) Umsetzung einer Verbindung der Formel 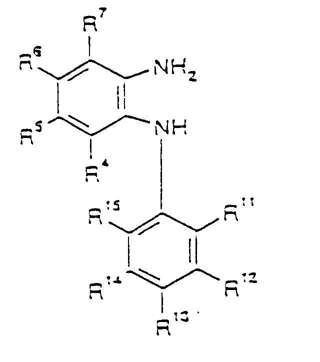 worin
R⁴, R⁵, R⁶, R⁷, R¹¹, R¹², R¹³, R¹⁴ und R¹⁵ die oben definierten Bedeutungen haben,
mit Cyanogenbromid zur Bildung einer Verbindung der Erfindung.

11. Verfahren nach Anspruch 10, wobei 1-(2-Hydroxy-5-phenyl-phenyl)-2-amino-benzimidazol hergestellt wird.

12. Verfahren nach Anspruch 10, wobei 1-(2-Methoxy-5-phenyl-phenyl)-2-amino-benzimidazol hergestellt wird.

13. Verfahren nach Anspruch 10, wobei 1-(2-Methoxy-5-phenyl-phenyl)-2-amino-5-trifluormethyl-benzimidazol hergestellt wird.

14. Verfahren nach Anspruch 10, wobei 1-(3-(1-Piperidyl)-phenyl)-5-fluor-2-amino-benzimidazol hergestellt wird.

## Revendications (Revendications pour l'(les) Etat(s) contractant(s) suivant(s): AT, BE, CH, LI, DE, DK, FR, GB, IT, LU, MC, NL, PT, SE)

1. Utilisation d'un composé ayant la formule 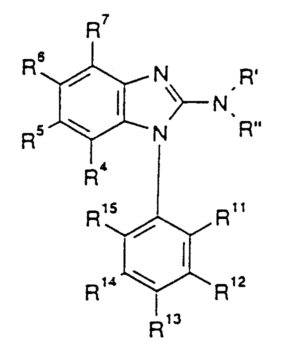 dans laquelle
R' et R'' sont indépendamment l'hydrogène ou un groupe alkyle en C₁ à C₄ qui peut être linéaire ou ramifié, ou bien R' et R'' forment ensemble une chaîne alkylène de 2 à 6 atomes de carbone ;
R⁴, R⁵, R⁶, R⁷, indépendamment les uns des autres, sont l'hydrogène, un halogène, CF₃ ou CN ;
R¹¹ est l'hydrogène, un halogène, CF₃, un groupe hydroxy ou ORⁱ où Rⁱ est un groupe alkyle en C₁ à C₄ qui peut être linéaire ou ramifié ;
R¹², R¹³ et R¹⁴ sont indépendamment l'hydrogène, un halogène, CF₃, un groupe alkyle en C₁ à C₄ qui peut être linéaire ou ramifié, OH, OR¹⁶ où R¹⁶ est un groupe alkyle en C₁ à C₄ qui peut être linéaire ou ramifié, un groupe phényle, pipéridyle, pyrrolidyle, ou phényle qui peut être substitué une ou plusieurs fois par un halogène, CF₃, CN, un groupe alkyle en C₁ à C₄ qui peut être linéaire ou ramifie, OH, NO₂, CO₂H, NH₂, ORⁱⁱ où Rⁱⁱ est un groupe alkyle en C₁ à C₄ qui peut être linéaire ou ramifié, CO₂Rⁱⁱⁱ où Rⁱⁱⁱ est un groupe alkyle en C₁ à C₄ qui peut être linéaire ou ramifié, NR^{iv}R^{v} où R^{iv} et R^{v} sont indépendamment l'hydrogène, un groupe alkyle en C₁ à C₆ qui peut être linéaire ou ramifié, un groupe acyle, ou dans lequel R^{iv} et R^{v} forment ensemble une chaîne alkylène de 2 à 6 atomes de carbone ; R¹⁵ est l'hydrogène ou, avec R¹⁴, forme un cycle benzénique supplémentaire ; ou un sel d'addition de celui-ci pharmaceutiquement acceptable, pour la préparation d'un médicament utile dans le traitement de troubles d'un mammifère, incluant un humain, réagissant au blocage des canaux calciques de type N et/ou de type L.

2. Utilisation d'un composé selon la revendication 1, pour la préparation d'un médicament utile dans le traitement de l'anoxie, de l'ischémie, de la migraine, de l'épilepsie, de la psychose, du parkinsonisme, de la dépression, et la prévention de changements dégénératifs liés avec l'anoxie, l'ischémie, la migraine et l'épilepsie.

3. Composé ayant la formule 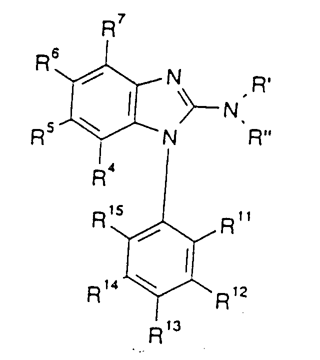 dans laquelle
R' et R'' sont indépendamment l'hydrogène ou un groupe alkyle en C₁ à C₄ qui peut être linéaire ou ramifié, ou bien R' et R'' forment ensemble une chaîne alkylène de 2 à 6 atomes de carbone ;
R⁴, R⁵, R⁶ et R⁷, indépendamment les uns des autres, sont l'hydrogène, un halogène, CF₃ ou CN ;
R¹¹ est l'hydrogène, un halogène, CF₃, un groupe hydroxy ou ORⁱ où Rⁱ est un groupe alkyle en C₁ à C₄ qui peut être linéaire ou ramifié ;
deux parmi R¹², R¹³ et R¹⁴ sont indépendamment l'hydrogène, un halogène, CF₃, un groupe alkyle en C₁ à C₄ qui peut être linéaire ou ramifié, OH, OR¹⁶ où R¹⁶ est un groupe alkyle en C₁ à C₄ qui peut être linéaire ou ramifié, ou un groupe phényle, et le dernier parmi R¹², R¹³ et R¹⁴ est un groupe pyrrolidyle, pipéridyle, ou phényle qui peut être substitué une ou plusieurs fois par un halogène, CF₃, CN, un groupe alkyle en C₁ à C₄ qui peut être linéaire ou ramifié, OH, NO₂, CO₂H, NH₂, ORⁱⁱ où Rⁱⁱ est un groupe alkyle en C₁ à C₄ qui peut être linéaire ou ramifié, CO₂Rⁱⁱⁱ où Rⁱⁱⁱ est un groupe alkyle en C₁ à C₄ qui peut être linéaire ou ramifié, NR^{iv}R^{v} où R^{iv} et R^{v} sont indépendamment l'hydrogène, un groupe alkyle en C₁ à C₆ qui peut être linéaire ou ramifié, un groupe acyle, ou dans lequel R^{iv} et R^{v} forment ensemble une chaîne alkylène de 2 à 6 atomes de carbone ; R¹⁵ est l'hydrogène ou, avec R¹⁴, forme un cycle benzénique supplémentaire ; ou un sel d'addition de celui-ci pharmaceutiquement acceptable.

4. Utilisation d'un composé selon la revendication 3, pour la préparation d'un médicament utile dans le traitement de troubles d'un mammifère, incluant un humain, réagissant au blocage des canaux calciques de type N et/ou de type L.

5. Composé selon la revendication 3, qui est le 1-(2-hydroxy-5-phényl-phényl)-2-aminobenzimidazole.

6. Composé selon la revendication 3, qui est le 1-(2-méthoxy-5-phényl-phényl)-2-aminobenzimidazole.

7. Composé selon la revendication 3, qui est le 1-(2-méthoxy-5-phényl-phényl)-2-amino-5-trifluorométhyl-benzimidazole.

8. Composé selon la revendication 3, qui est le 1-(3-(1-pipéridyl)-phényl)-5-fluoro-2-aminobenzimidazole.

9. Composition pharmaceutique comprenant, en tant qu'ingrédient actif, une quantité efficace d'un composé de la revendication 3.

10. Procédé de préparation d'un composé selon la revendication 3, comprenant :
a) la réaction d'un composé ayant la formule 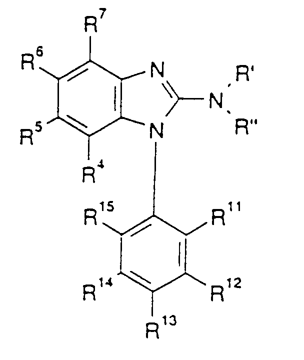 dans laquelle,
R', R'', R⁴, R⁵, R⁶, R⁷ et R¹⁵ ont les significations présentées ci-dessus et dans laquelle l'un parmi R¹², R¹³ et R¹⁴ est de l'iode et les autres parmi R¹², R¹³ et R¹⁴ ont les significations présentées ci-dessus, avec R¹²-B(OH)₂, R¹³-B(OH)₂ ou R¹⁴-B(OH)₂, où R¹², R¹³ et R¹⁴ ont les significations présentées ci-dessus, pour former un composé de l'invention, ou
b) la réaction d'un composé ayant la formule 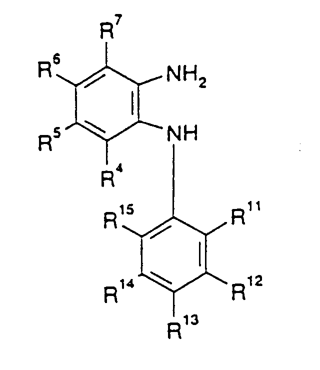 dans laquelle,
R⁴, R⁵, R⁶, R⁷, R¹¹, R¹², R¹³, R¹⁴ et R¹⁵ ont les significations présentées ci-dessus, avec du bromure de cyanogène, pour former un composé de l'invention.

11. Procédé selon la revendication 10, dans lequel du 1-(2-hydroxy-5-phényl-phényl)-2-aminobenzimidazole est préparé.

12. Procédé selon la revendication 10, dans lequel du 1-(2-méthoxy-5-phényl-phényl)-2-aminobenzimidazole est préparé.

13. Procédé selon la revendication 10, dans lequel du 1-(2-méthoxy-5-phényl-phényl)-2-amino-5-trifluorométhyl-benzimidazole est préparé.

14. Procédé selon la revendication 10, dans lequel du 1-(3-(1-pipéridyl)-phényl)-5-fluoro-2-aminobenzimidazole est préparé.

## Revendications (Revendications pour l'(les) Etat(s) contractant(s) suivant(s): ES)

1. Composé ayant la formule 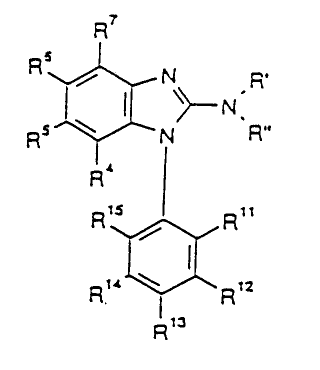 dans laquelle
R' et R'' sont indépendamment l'hydrogène ou un groupe alkyle en C₁ à C₄ qui peut être linéaire ou ramifié, ou bien R' et R'' forment ensemble une chaîne alkylène de 2 à 6 atomes de carbone ;
R⁴, R⁵, R⁶ et R⁷, indépendamment les uns des autres, sont l'hydrogène, un halogène, CF₃ ou CN ;
R¹¹ est l'hydrogène, un halogène, CF₃, un groupe hydroxy ou ORⁱ où Rⁱ est un groupe alkyle en C₁ à C₄ qui peut être linéaire ou ramifié ;
deux parmi R¹², R¹³ et R¹⁴ sont indépendamment l'hydrogène, un halogène, CF₃, un groupe alkyle en C₁ à C₄ qui peut être linéaire ou ramifié, OH, OR¹⁶ où R¹⁶ est un groupe alkyle en C₁ à C₄ qui peut être linéaire ou ramifié, ou un groupe phényle, et le dernier de R¹², R¹³ et R¹⁴ est un groupe pyrrolidyle, pipéridyle, ou phényle qui peut être substitué une ou plusieurs fois par un halogène, CF₃, CN, un groupe alkyle en C₁ à C₄ qui peut être linéaire ou ramifié, OH, NO₂, CO₂H, NH₂, ORⁱⁱ où Rⁱⁱ est un groupe alkyle en C₁ à C₄ qui peut être linéaire ou ramifié, CO₂Rⁱⁱⁱ où Rⁱⁱⁱ est un groupe alkyle on C₁ à C₄ qui peut être linéaire ou ramifié, NR^{iv}R^{v} où R^{iv} et R^{v} sont indépendamment l'hydrogène, un groupe alkyle en C₁ à C₆ qui peut être linéaire ou ramifié, un groupe acyle, ou dans lequel R^{iv} et R^{v} forment ensemble une chaîne alkylène de 2 à 6 atomes de carbone ; R¹⁵ est l'hydrogène ou, avec R¹⁴, forme un cycle benzénique supplémentaire ; ou un sel d'addition de celui-ci pharmaceutiquement acceptable.

2. Composé selon la revendication 1, qui est le 1-(2-hydroxy-5-phényl-phényl)-2-aminobenzimidazole.

3. Composé selon la revendication 1, qui est le 1-(2-méthoxy-5-phényl-phényl)-2-aminobenzimidazole.

4. Composé selon la revendication 1, qui est le 1-(2-méthoxy-5-phényl-phényl)-2-amino-5-trifluorométhyl-benzimidazole.

5. Composé selon la revendication 1, qui est le 1-(3-(1-pipéridyl)-phényl)-5-fluoro-2-aminobenzimidazole.

6. Procédé pour la préparation d'une composition pharmaceutique comprenant, en tant qu'ingrédient actif, une quantité efficace d'un composé de la revendication 1, le procédé comprenant l'étape consistant à mettre le composé, avec un adjuvant, véhicule ou diluant classique, sous la forme d'une composition pharmaceutique.

7. Procédé de préparation d'un composé selon la revendication 1, comprenant :
a) la réaction d'un composé ayant la formule 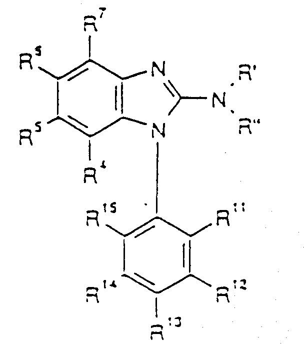 dans laquelle,
R', R'', R⁴, R⁵, R⁶, R⁷ et R¹⁵ ont les significations présentées ci-dessus et dans laquelle l'un parmi R¹², R¹³ et R¹⁴ est de l'iode et les autres parmi R¹², R¹³ et R¹⁴ ont les significations présentées ci-dessus, avec R¹²-B(OH)₂, R¹³-B(OH)₂ ou R¹⁴-B(OH)2, où R¹², R¹³ et R¹⁴ ont les significations présentées ci-dessus, pour former un composé de l'invention, ou
b) la réaction d'un composé ayant la formule 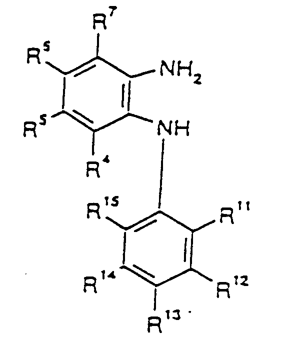 dans laquelle,
R⁴, R⁵, R⁶, R⁷, R¹¹, R¹², R¹³, R¹⁴ et R¹⁵ ont les significations présentées ci-dessus, avec du bromure de cyanogène, pour former un composé de l'invention.

8. Procédé selon la revendication 7, dans lequel du 1-(2-hydroxy-5-phényl-phényl)-2-aminobenzimidazole est préparé.

9. Procédé selon la revendication 7, dans lequel du 1-(2-méthoxy-5-phényl-phényl)-2-aminobenzimidazole est préparé.

10. Procédé selon la revendication 7, dans lequel du 1-(2-méthoxy-5-phényl-phényl)-2-amino-5-trifluorométhyl-benzimidazole est préparé.

11. Procédé selon la revendication 7, dans lequel du 1-(3-(1-pipéridyl)-phényl)-5-fluoro-2-aminobenzimidazole est préparé.

## Revendications (Revendications pour l'(les) Etat(s) contractant(s) suivant(s): GR)

1. Utilisation d'un composé ayant la formule 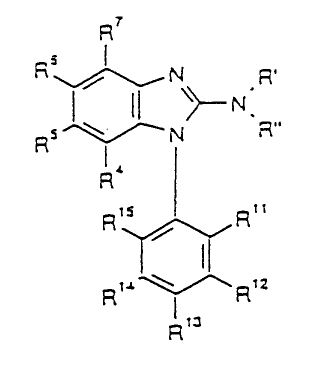 dans laquelle
R' et R'' sont indépendamment l'hydrogène ou un groupe alkyle en C₁ à C₄ qui peut être linéaire ou ramifié, ou bien R' et R'' forment ensemble une chaîne alkylène de 2 à 6 atomes de carbone ;
R⁴, R⁵, R⁶, R⁷, indépendamment les uns des autres, sont l'hydrogène, un halogène, CF₃ ou CN ;
R¹¹ est l'hydrogène, un halogène, CF₃, un groupe hydroxy ou ORⁱ où Rⁱ est un groupe alkyle en C₁ à C₄ qui peut être linéaire ou ramifié ;
R¹², R¹³ et R¹⁴ sont indépendamment l, hydrogène, un halogène, CF₃, un groupe alkyle en C₁ à C₄ qui peut être linéaire ou ramifié, OH, OR¹⁶ où R¹⁶ est un groupe alkyle en C₁ à C₄ qui peut être linéaire ou ramifié, un groupe phényle, pipéridyle, pyrrolidyle, ou phényle qui peut être substitué une ou plusieurs fois par un halogène, CF₃, CN, un groupe alkyle en C₁ à C₄ qui peut être linéaire ou ramifié, OH, NO₂, CO₂H, NH₂, ORⁱⁱ où Rⁱⁱ est un groupe alkyle en C₁ à C₄ qui peut être linéaire ou ramifié, CO₂Rⁱⁱⁱ où Rⁱⁱⁱ est un groupe alkyle en C₁ à C₄ qui peur être linéaire ou ramifié, NR^{iv}R^{v} où R^{iv} et R^{v} sont indépendamment l'hydrogène, un groupe alkyle en C₁ à C₆ qui peut être linéaire ou ramifié, un groupe acyle, ou dans lequel R^{iv} et R^{v} forment ensemble une chaîne alkylène de 2 à 6 atomes de carbone ; R¹⁵ est l'hydrogène ou, avec R¹⁴, forme un cycle benzénique supplémentaire ; ou un sel d'addition de celui-ci pharmaceutiquement acceptable, pour la préparation d'un médicament utile dans le traitement de troubles d'un mammifère, incluant un humain, réagissant au blocage des canaux calciques de type N et/ou de type L.

2. Utilisation d'un composé selon la revendication 1, pour la préparation d'un médicament utile dans le traitement de l'anoxie, de l'ischémie, de la migraine, de l'épilepsie, de la psychose, du parkinsonisme, de la dépression, et la prévention de changements dégénératifs liés avec l'anoxie, l'ischémie, la migraine et l'épilepsie.

3. Composé ayant la formule 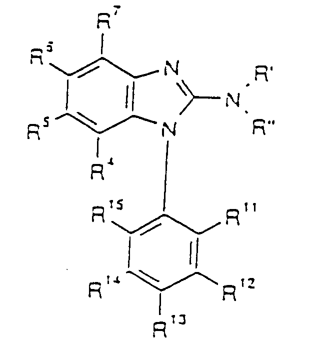 dans laquelle
R' et R'' sont indépendamment l'hydrogène ou un groupe alkyle en C₁ à C₄ qui peut être linéaire ou ramifié, ou bien R' et R'' forment ensemble une chaîne alkylène de 2 à 6 atomes de carbone ;
R⁴, R⁵, R⁶ et R⁷, indépendamment les uns des autres, sont l'hydrogène, un halogène, CF₃ ou CN ;
R¹¹ est l'hydrogène, un halogène, CF₃, un groupe hydroxy ou ORⁱ où Rⁱ est un groupe alkyle en C₁ à C₄ qui peut être linéaire ou ramifié ;
deux parmi R¹², R¹³ et R¹⁴ sont indépendamment l'hydrogène, un halogène, CF₃, un groupe alkyle en C₁ à C₄ qui peut être linéaire ou ramifié, OH, OR¹⁶ où R¹⁶ est un groupe alkyle en C₁ à C₄ qui peut être linéaire ou ramifié, ou un groupe phényle, et le dernier parmi R¹², R¹³ et R¹⁴ est un groupe pyrrolidyle, pipéridyle, ou phényle qui peut être substitué une ou plusieurs fois par un halogène, CF₃, CN, un groupe alkyle en C₁ à C₄ qui peut être linéaire ou ramifié, OH, NO₂, CO₂H, NH₂, ORⁱⁱ où Rⁱⁱ est un groupe alkyle en C₁ à C₄ qui peut être linéaire ou ramifié, CO₂Rⁱⁱⁱ où Rⁱⁱⁱ est un groupe alkyle en C₁ à C₄ qui peut être linéaire ou ramifié, NR^{iv}R^{v} où R^{iv} et R^{v} sont indépendamment l'hydrogène, un groupe alkyle en C₁ à C₆ qui peut être linéaire ou ramifié, un groupe acyle, ou dans lequel R^{iv} et R^{v} forment ensemble une chaîne alkylène de 2 à 6 atomes de carbone ; R¹⁵ est l'hydrogène ou, avec R¹⁴, forme un cycle benzénique supplémentaire ; ou un sel d'addition de celui-ci pharmaceutiquement acceptable.

4. Utilisation d'un composé selon la revendication 3, pour la préparation d'un médicament utile dans le traitement de troubles d'un mammifère, incluant un humain, réagissant au blocage des canaux calciques de type N et/ou de type L.

5. Composé selon la revendication 3, qui est le 1-(2-hydroxy-5-phényl-phényl)-2-aminobenzimidazole.

6. Composé selon la revendication 3, qui est le 1-(2-méthoxy-5-phényl-phényl)-2-aminobenzimidazole.

7. Composé selon la revendication 3, qui est le 1-(2-méthoxy-5-phényl-phényl)-2-amino-5-trifluorométhyl-benzimidazole.

8. Composé selon la revendication 3, qui est le 1-(3-(1-pipéridyl)-phényl)-5-fluoro-2-aminobenzimidazole.

9. Procédé pour la préparation d'une composition pharmaceutique comprenant, en tant qu'ingrédient actif, une quantité efficace d'un composé de la revendication 3, le procédé comprenant l'étape consistant à mettre le composé, avec un adjuvant, véhicule ou diluant classique, sous la forme d'une composition pharmaceutique.

10. Procédé de préparation d'un composé selon la revendication 3, comprenant :
a) la réaction d'un composé ayant la formule 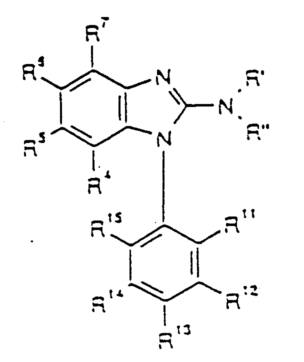 dans laquelle,
R', R'', R⁴, R⁵, R⁶, R⁷ et R¹⁵ ont les significations présentées ci-dessus et dans laquelle l'un parmi R¹², R¹³ et R¹⁴ est de l'iode et les autres parmi R¹², R¹³ et R¹⁴ ont les significations présentées ci-dessus, avec R¹²-B(OH)₂, R¹³-B(OH)₂ ou R¹⁴-B(OH)₂, où R¹², R¹³ et R¹⁴ ont les significations présentées ci-dessus, pour former un composé de l'invention, ou
b) la réaction d'un composé ayant la formule 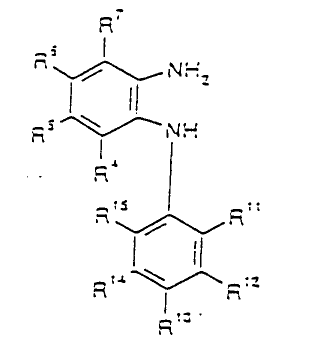 dans laquelle,
R⁴, R⁵, R⁶, R⁷, R¹¹, R¹², R¹³, R¹⁴ et R¹⁵ ont les significations présentées ci-dessus, avec du bromure de cyanogène, pour former un composé de l'invention.

11. Procédé selon la revendication 10, dans lequel du 1-(2-hydroxy-5-phényl-phényl)-2-aminobenzimidazole est préparé.

12. Procédé selon la revendication 10, dans lequel du 1-(2-méthoxy-5-phényl-phényl)-2-aminobenzimidazole est préparé.

13. Procédé selon la revendication 10, dans lequel du 1-(2-méthoxy-5-phényl-phényl)-2-amino-5-trifluorométhyl-benzimidazole est préparé.

14. Procédé selon la revendication 10, dans lequel du 1-(3-(1-pipéridyl)-phényl)-5-fluoro-2-aminobenzimidazole est préparé.
